# EUROPEAN PATENT APPLICATION

(11) **EP 2 161 252 A1**
(43) Date of publication of application: **10.03.2010**
(21) Application number: 09165292.5
(22) Date of filing: 23.03.2006
(51) Int. Cl.: C07C 233/63, C07D 307/32, A61P 11/14, A61P 25/34, A61P 17/00, A61K 31/223, A61K 31/365

(54) **N-Alkylcarbonyl-amino acid ester and N-Alkylcarbonyl-amino lactone compounds and their use**

(30) Priority: 29.03.2005 US 667166 P; 20.05.2005 US 683384 P; 25.07.2005 US 702505 P; 13.08.2005 US 203728; 09.02.2006 US 772374 P
(62) Divisional of application: 06710144.4
(71) Applicant: Wei, Edward T., Berkeley, CA 94708 (US)
(72) Inventor: Wei, Edward T., Berkeley, CA 94708 (US)
(74) Representative: Florence, Julia Anne

(57) **Abstract**

The present invention generally relates to refreshing, soothing, and cooling compounds that affect sensory processes. More particularly, the present invention pertains to certain *N*-alkylcarbonyl-amino acid ester and *N*-alkylcarbonyl-amino lactone compounds as described herein; compositions and articles comprising such compounds; and methods of treatment, for example, methods of alleviating the discomforts of irritation, itch, and pain in the skin and in the linings of the oral cavity and upper respiratory tract, for example, in methods of treatment of cough and/or asthma.

## Description

### RELATED APPLICATIONS

This application is related to:
U.S. Provisional Application No. 60/667,166 filed 29 March 2005;
U.S. Provisional Application No. 60/683,384 filed 20 May 2005;
U.S. Provisional Application No. 60/702,505 filed 26 July 2005;
U.S. Patent Application No. 11/203,728 filed 13 August 2005; and
U.S. Provisional Application No. 60/772,374 filed 09 February 2006;
the contents of each of which are incorporated herein by reference in their entirety.

### TECHNICAL FIELD

The present invention generally relates to refreshing, soothing, and cooling compounds that affect sensory processes. More particularly, the present invention pertains to certain *N*-alkylcarbonyl-amino acid ester and *N*-alkylcarbonyl-amino lactone compounds; compositions and articles comprising such compounds; and methods of treatment, for example, methods of alleviating the discomforts of irritation, itch, and pain in the skin and in the linings of the oral cavity and upper respiratory tract, for example, in methods of treatment of cough and/or asthma.

### BACKGROUND

A number of patents and publications are cited herein in order to more fully describe and disclose the invention and the state of the art to which the invention pertains. Each of these references is incorporated herein by reference in its entirety into the present disclosure, to the same extent as if each individual reference was specifically and individually indicated to be incorporated by reference.

Throughout this specification, including the claims which follow, unless the context requires otherwise, the word "comprise," and variations such as "comprises" and "comprising," will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Thus, for example, reference to "a pharmaceutical carrier" includes mixtures of two or more such carriers, and the like.

Ranges are often expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment.

Menthol and menthol-like compounds are used in toiletries, confectionery, comestibles, and over-the-counter medications as ingredients to refresh, cool, flavor, counter-irritate, and anesthetize the skin and mucous membranes of the mouth and upper airways. Menthol's utility in relief of sensory discomfort is, however, limited by its short duration of action and by its multimodal actions on sensory processes - including odor, harshness of taste, and irritation. The unpleasant effects of menthol can be easily experienced, for example, when menthol-containing ointments are brought near the eye surface. The menthol vapors hurt the eye and causes tearing. Similarly, menthol can relieve nasal congestion and suppress cough - but the effect, especially for cough, is transient, and the irritant properties of mucous membranes limit the use of higher dosage.

Current treatments for the discomforts of injured skin include cold water rinses or compresses, and ointments containing local anesthetics (such as EMLA®), or non-steroidal anti-inflammatory analgesics (NSAIDs). Current medications for cough are dextrorphan, codeine, and menthol. These methods and compounds have moderate effectiveness and ease of use.

There is a need for compounds like menthol that refresh, cool, and soothe the body's surfaces, but without the disadvantages of odor, irritancy, and most importantly, a short duration of action. In order to treat medically important discomforts of the skin, such as pruritic eczema, or the sustained coughing and wheezing of asthma, it is important to have compounds that act much longer than menthol.

Menthol has three chiral centres, and all eight optical isomers are known. The most common optical isomer, denoted (-)-menthol (also: 1R-(1α-2β-5α)-5-methyl-2-(1-methylethyl)cyclohexanol) is shown below.

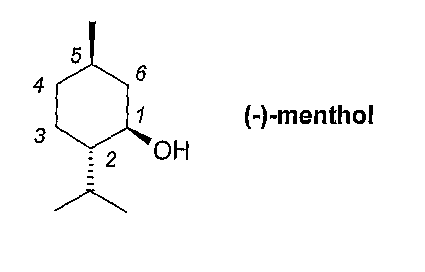

About three decades ago, a group of scientists synthesized over 1200 compounds in an attempt to find cooling agents that had properties better than menthol. Their results were summarized in a paper (Watson et al., "New compounds with the menthol cooling effect," J. Soc. Cosmet. Chem., 29: 185-200, 1978). From this research, an *N*-alkyl-cycloalkyl- and an *N*-alkyl-alkyl carboxamide, WS-3 and WS-23, were brought to the market and are used as additives for confectionery, comestibles, (e.g., candy, chewing gum), and toiletries.

In U.S. Patent No. 4,178,459 (11 December 1979), Watson et al. described cooling properties of some *N*-alkoxycarbonylalkyl-substituted p-menthane-carboxamides. An alanine ethyl ester compound (shown below) is indicated in the table appearing in columns 5 and 6. This designation is silent with respect to stereoisomerism, both on the menthane moiety and the alanine moiety.

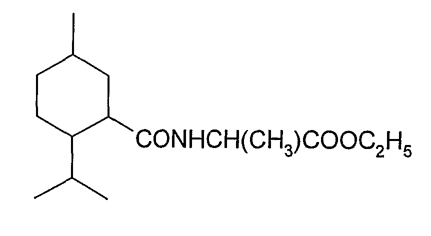

Other menthol-like cooling compounds in commercial use for applications to skin and mucous membranes are, for example, menthyl lactate (Frescolat® ML) and menthoxypropanediol (Cooling Agent 10).

The recent information on cooling agents used for topical applications was reviewed by M.B. Erman ("Cooling agents and skin care applications," Cosmetics & Toiletries, 120: 105-118, May 2005; "Progress in physiological cooling agents," Perfumer & Flavorist, 29: 34-50, 2004) and by P. Jacobs and W. Johncock ("Some like it cool," Parfumerie und Ksometik, 80: 26-31, 1999).

A glycine ethyl ester compound (shown below), known as WS-5, is shown in Figure F-11 on page 42 of the review article by Erman, mentioned above. This compound lacks an α-methyl group or α-ethyl group.

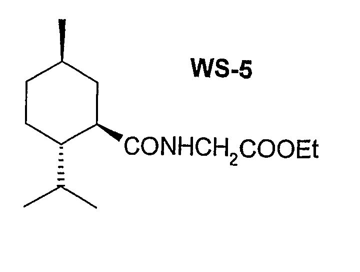

This compound (WS-5) and it's optical isomers are also the subject of U.S. Patent Publication No. 2005/0222256 A1 (06 October 2005).

None of the compounds currently known to the art have the potency or duration of action to qualify them as possible prescription medications for use in severe skin disorders such as pruritic eczema or in upper respiratory ailments such as asthma.

### SUMMARY OF THE INVENTION

One aspect of the present invention pertains to certain *N*-alkylcarbonyl-amino acid ester and *N*-alkylcarbonyl-amino lactone compounds, as described herein.

Another aspect of the invention pertains to a composition comprising such a compound and a delivery vehicle (e.g., for delivering the compound to a human).

In one embodiment, the delivery vehicle is a pharmaceutically acceptable carrier or diluent.

In one embodiment, the delivery vehicle is adapted to deliver the compound to the skin of the human.

In one embodiment, the delivery vehicle is a towelette.

In one embodiment, the delivery vehicle is adapted to deliver the compound to the oral cavity and/or the upper respiratory tract of the human.

In one embodiment, the compound is present in the composition in an amount of 1 to 10 mg, for example, in a lozenge.

In one embodiment, the compound is present in the composition in an amount of 0.01 to 2% wt/vol.

In one embodiment, the composition further comprises a polyhydric alcohol.

In one embodiment, the composition further comprises a mucoadhesive polymer.

Another aspect of the present invention pertains to methods of treatment of the skin, oral cavity, or upper airways of a human, comprising:
contacting a composition comprising such a compound and a delivery vehicle with the skin, oral cavity, or upper airways of the human, thereby delivering an effective amount of the compound to the skin or mucous membranes of the human.

Another aspect of the present invention pertains to methods of treatment of a condition, comprising:
contacting a composition comprising a compound according to any one of claims 1 to 24 and a delivery vehicle with, e.g., the skin, oral cavity, or upper airways of the human,
thereby delivering an amount of the compound therapeutically effective for treatment of (e.g., alleviation of) the condition.

Another aspect of the present invention pertains to such a compound for use in a method of treatment of the human or animal body by therapy.

Another aspect of the present invention pertains to use of such a compound in the manufacture of a medicament for use in a method of treatment.

In one embodiment, the treatment is treatment of (e.g., alleviation of) skin irritation, itch, and/or pain (e.g., wherein the contacting delivers an amount of the compound that is therapeutically effective for alleviation of skin irritation, itch, and/or pain).

In one embodiment, the treatment is treatment of (e.g., alleviation of) cough and/or the sense of irritation and/or obstruction of the upper airways (e.g., wherein the contacting delivers an amount of the compound that is therapeutically effective for alleviation of cough and/or the sense of irritation and/or obstruction of the upper airways).

In one embodiment, the treatment is treatment of (e.g., alleviation of) the symptoms and signs of asthma, chronic obstructive pulmonary disease, or other disease of the upper airways (e.g., wherein the contacting delivers an amount of the compound that is therapeutically effective for alleviation of the symptoms and signs of asthma, chronic obstructive pulmonary disease, or other disease of the upper airways).

In one embodiment, the treatment is treatment of cough (e.g., wherein the contacting delivers an amount of the compound that is therapeutically effective for treatment of cough).

In one embodiment, the treatment is treatment of asthma (e.g., wherein the contacting delivers an amount of the compound that is therapeutically effective for treatment of asthma).

In one embodiment, the treatment is smoking cessation therapy (e.g., wherein the contacting delivers an amount of the compound that is effective in smoking cessation therapy).

In one embodiment, the treatment is treatment to reduce host dissemination of an infectious microorganism (e.g., wherein the contacting delivers an amount of the compound that is effective to reduce host dissemination of an infectious microorganism).

In one embodiment, the treatment is treatment to prevent coughing and airborne transmission of an infectious microorganism (e.g., wherein the contacting delivers an amount of the compound that is effective to prevent coughing and airborne transmission of an infectious microorganism).

In one embodiment, the treatment is treatment to increase alertness, or to decrease nausea, appetite, fatigue, heat, or fever (e.g., wherein the contacting delivers an amount of the compound that is effective to increase alertness, or to decrease nausea, appetite, fatigue, heat, or fever).

As will be appreciated by one of skill in the art, features and preferred embodiments of one aspect of the invention will also pertain to other aspect of the invention.

Other advantages and aspects of the invention will be understood by reading the following detailed description and the accompanying claims.

### BRIEF DESCRIPTION OF THE DRAWINGS

Figure 1 is a graph of cooling intensity versus time (hours) after application for six compounds: WS-3 (filled circles), (-)-menthol (open circles), WS-5 (filled triangles), D-Hsl (open triangles), D-Ala-OMe (filled squares), and D-Ala-OEt (open squares).
Figure 2 is a bar graph showing duration of cooling (hours) for eight compounds (in order): D-Ala-OEt, D-Ala-OMe, D-Hsl, Sar-OEt, WS-5, WS-12, WS-3, and (-)-menthol.

### DETAILED DESCRIPTION

A class of compounds that is suitable to be used as an active ingredient in (e.g., pharmaceutical) preparations for use on skin, lips, and mucous membranes of the oral cavity and upper respiratory tract has been found.

These compounds are suitable, for example, for use as therapeutic agents, to reduce discomfort such as itch and pain, and as additives for comestibles, confectionery, cosmetics, and toiletries.

These compounds have one or more of the following properties:
(i) a refreshing, soothing, and cooling action on surfaces of the skin, oral cavity, and/or throat, and, in pathological states, an anti-irritant, anti-pruritic, anti-tussive, and/or anti-nociceptive effect;
(ii) a minimal irritant action on the eye when the compound is applied to facial skin near and around the eyes, for example, when applied to the malar and periorbital skin (indicating also a good safety profile), for example, when applied on the skin at a concentration of 40 mM or less, equivalent to a 1% wt/vol mixture for a molecule of 250 Daltons;
(iii) a rapid onset of action of less than about 5 minutes (e.g., from 0.5 to 5 minutes), preferably less than about 3 minutes (e.g., from 0.5 to 3 minutes), preferably less than about 1 minute (e.g., from 0.5 to 1 minute), for example, when applied on the skin at a concentration of 40 mM or less, equivalent to a 1% wt/vol mixture for a molecule of 250 Daltons;
(iv) a duration of action that exceeds 1 hour, for example, when applied on the skin at a concentration of 40 mM or less, equivalent to a 1% wt/vol mixture for a molecule of 250 Daltons;
(v) wherein repeat applications do not result in altered sensitivity to subsequent stimulation; and
(vi) a potent cool, soothing, and refreshing sensation when applied into the oral cavity that counteracts irritative stimuli in the mouth and upper airways that causes cough and wheezing.

These compounds may conveniently be referred to as *N*-alkylcarbonyl- and *N*-alkyl-*N-*alkylcarbonyl- D-, L-, or DL- amino acid esters or lactones, or "NACE compounds".

In one embodiment, the compound is selected from compounds of Formula (1): 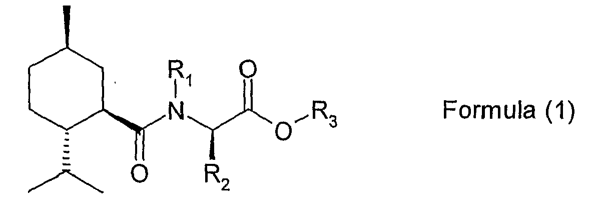 wherein:
R₁ is independently hydrogen or methyl;
R₂ is independently C₁ to C₂ alkyl; and
R₃ is independently C₁ to C₄ alkyl.

The menthyl group (i.e., the 5-methyl-2-(1-methylethyl)cyclohex-1-yl group) has the same stereochemistry as found in (-)-menthol.

The α-carbon, between the -NR₁- group and the -C(=O)OR₃ group, has the same stereochemistry as found in D-alanine. This is also known as the *R*-configuration, according to the Cahn-Ingold-Prelog nomenclature system. Except for glycine, all α-amino acids have a chiral center at the α-carbon. Although amino acids of the D-configuration are found in some antibiotics and in cell membranes of microorganisms, the amino acids of proteins are (almost) exclusively of the L (or S configuration).

In one embodiment, R₁ is independently hydrogen.
In one embodiment, R₁ is independently methyl.

In one embodiment, R₂ is independently methyl.
In one embodiment, R₂ is independently ethyl.

In one embodiment, R₃ is independently methyl, ethyl, *n*-propyl, *i*-propyl, *n*-butyl, *i*-butyl, *s*-butyl, or *t*-butyl.
In one embodiment, R₃ is independently methyl or ethyl.
In one embodiment, R₃ is independently methyl.
In one embodiment, R₃ is independently ethyl.

Each and every compatible combination of the embodiments described above is explicitly disclosed herein, as if each and every combination was individually and explicitly recited.

The combination of R₂ and the D-configuration has the effect of increasing potency and duration of action, and of producing a selective refreshing coolness with the absence of tissue irritation.

In a preferred embodiment, R₁ is hydrogen, R₂ is methyl, and R₃ is methyl or ethyl (giving the corresponding D-alanyl methyl ester or D-alanyl methyl ethyl ester derivatives).

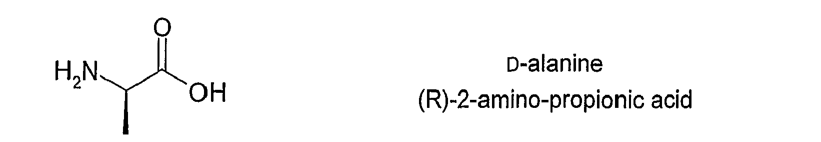

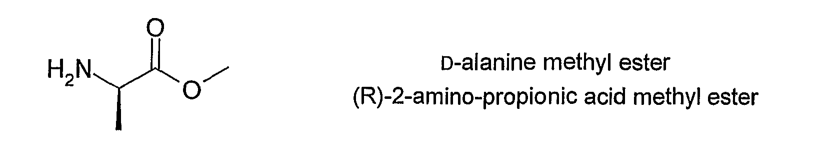

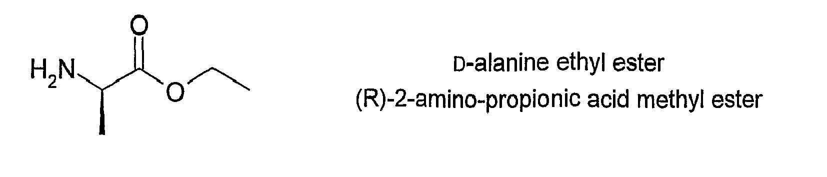

Examples of some preferred compounds include the following:

| | | |
|---|---|---|
| 1 | (R)-2-[((1R,2S,5R)-2-isopropyl-5-methyl-cyclohexanecarbonyl)-amino]-propionic acid methyl ester | 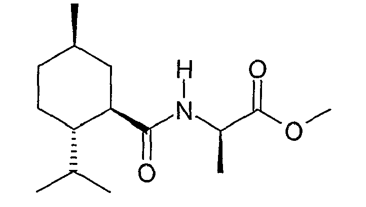 |
| 2 | (R)-2-[((1R,2S,5R)-2-isopropyl-5-methyl-cyclohexanecarbonyl)-amino]-propionic acid ethyl ester | 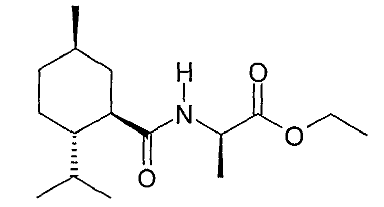 |
| 3 | (R)-2-[((1R,2S,5R)-2-isopropyl-5-methyl-cyclohexanecarbonyl)-amino]-propionic acid *n*-propyl ester | 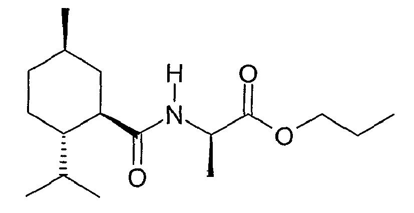 |
| 4 | (R)-2-[((1R,2S,5R)-2-isopropyl-5-methyl-cyclohexanecarbonyl)-amino]-propionic acid *i*-propyl ester | 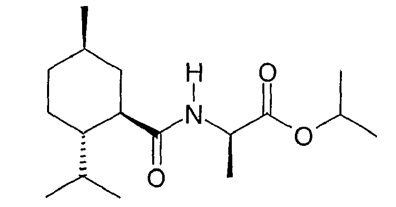 |
| 5 | (R)-2-[((1R,2S,5R)-2-isopropyl-5-methyl-cyclohexanecarbonyl)-amino]-propionic acid *n*-butyl ester | 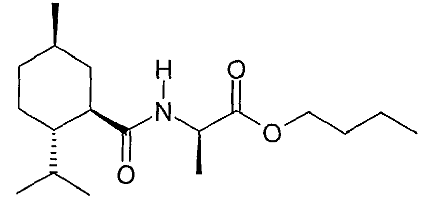 |
| 6 | (R)-2-[((1R,2S,5R)-2-isopropyl-5-methyl-cyclohexanecarbonyl)-amino]-propionic acid *sec*-butyl ester | 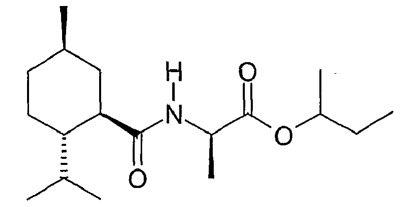 |
| 7 | (R)-2-[((1R,2S,5R)-2-isopropyl-5-methyl-cyclohexanecarbonyl)-amino]-propionic acid *i*-butyl ester | 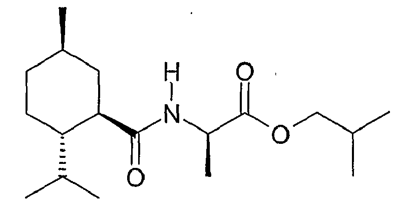 |
| 8 | (R)-2-[((1R,2S,5R)-2-isopropyl-5-methyl-cyclohexanecarbonyl)-amino]-propionic acid *t*-butyl ester | 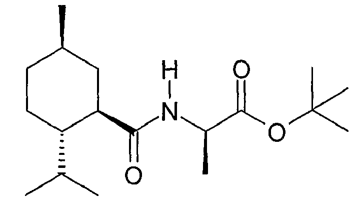 |

Additional examples of preferred compounds include the following:

| | |
|---|---|
| 1 | (R)-2-[((1R,2S,5R)-2-isopropyl-5-methyl-cyclohexanecarbonyl)-methyl-amino]-propionic acid methyl ester |
| 2 | (R)-2-[((1R,2S,5R)-2-isopropyl-5-methyl-cyclohexanecarbonyl)-methyl-amino]-propionic acid ethyl ester |
| 3 | (R)-2-[((1R,2S,5R)-2-isopropyl-5-methyl-cyclohexanecarbonyl)-methyl-amino]-propionic acid *n*-propyl ester |
| 4 | (R)-2-[((1R,2S,5R)-2-isopropyl-5-methyl-cyclohexanecarbonyl)-methyl-amino]-propionic acid *i*-propyl ester |
| 5 | (R)-2-[((1R,2S,5R)-2-isopropyl-5-methyl-cyclohexanecarbonyl)-methyl-amino]-propionic acid *n*-butyl ester |
| 6 | (R)-2-[((1R,2S,5R)-2-isopropyl-5-methyl-cyclohexanecarbonyl)-methyl-amino]-propionic acid *sec*-butyl ester |
| 7 | (R)-2-[((1R,2S,5R)-2-isopropyl-5-methyl-cyclohexanecarbonyl)-methyl-amino]-propionic acid *i*-butyl ester |
| 8 | (R)-2-[((1R,2S,5R)-2-isopropyl-5-methyl-cyclohexanecarbonyl)-methyl-amino]-propionic acid *t*-butyl ester |

Additional examples of preferred compounds include the following:

| | |
|---|---|
| 1 | (R)-2-[((1R,2S,5R)-2-isopropyl-5-methyl-cyclohexanecarbonyl)-aminoj-butyric acid methyl ester |
| 2 | (R)-2-[((1R,2S,5R)-2-isopropyl-5-methyl-cyclohexanecarbonyl)-amino]-butyric acid ethyl ester |
| 3 | (R)-2-[((1R,2S,5R)-2-isopropyl-5-methyl-cyclohexanecarbonyl)-amino]-butyric acid *n*-propyl ester |
| 4 | (R)-2-[((1R,2S,5R)-2-isopropyl-5-methyl-cyclohexanecarbonyl)-amino]-butyric acid *i*-propyl ester |
| 5 | (R)-2-[((1R,2S,5R)-2-isopropyl-5-methyl-cyclohexanecarbonyl)-amino]-butyric acid *n*-butyl ester |
| 6 | (R)-2-[((1R,2S,5R)-2-isopropyl-5-methyl-cyclohexanecarbonyl)-amino]-butyric acid *sec*-butyl ester |
| 7 | (R)-2-[((1R,2S,5R)-2-isopropyl-5-methyl-cyclohexanecarbonyl)-amino]-butyric acid *i*-butyl ester |
| 8 | (R)-2-[((1R,2S,5R)-2-isopropyl-5-methyl-cyclohexanecarbonyl)-amino]-butyric acid *t*-butyl ester |

Additional examples of preferred compounds include the following:

| | |
|---|---|
| 1 | (R)-2-[((1R,2S,5R)-2-isopropyl-5-methyl-cyclohexanecarbonyl)-methyl-amino]-butyric acid methyl ester |
| 2 | (R)-2-[((1R,2S,5R)-2-isopropyl-5-methyl-cyclohexanecarbonyl)-methyl-amino]-butyric acid ethyl ester |
| 3 | (R)-2-[((1R,2S,5R)-2-isopropyl-5-methyl-cyclohexanecarbonyl)-methyl-amino]-butyric acid *n*-propyl ester |
| 4 | (R)-2-[((1R,2S,5R)-2-isopropyl-5-methyl-cyclohexanecarbonyl)-methyl-amino]-butyric acid *i*-propyl ester |
| 5 | (R)-2-[((1R,2S,5R)-2-isopropyl-5-methyl-cyclohexanecarbonyl)-methyl-amino]-butyric acid *n*-butyl ester |
| 6 | (R)-2-[((1R,2S,5R)-2-isopropyl-5-methyl-cyclohexanecarbonyl)-methyl-amino]-butyric acid *sec*-butyl ester |
| 7 | (R)-2-[((1R,2S,5R)-2-isopropyl-5-methyl-cyclohexanecarbonyl)-methyl-amino]-butyric acid *i*-butyl ester |
| 8 | (R)-2-[((1R,2S,5R)-2-isopropyl-5-methyl-cyclohexanecarbonyl)-methyl-amino]-butyric acid *t*-butyl ester |

In one embodiment, the compound is selected from compounds of Formula (2): 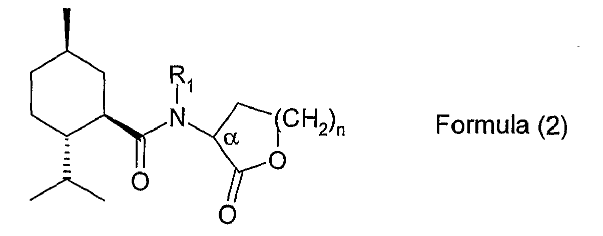 wherein:
n is independently 1, 2, or 3; and
R₁ is independently hydrogen or methyl.

Again, the menthyl group (i.e., the 5-methyl-2-(1-methylethyl)cyclohex-1-yl group) has the same stereochemistry as found in (-)-menthol.

In one embodiment, n is independently 1.
In one embodiment, n is independently 2.
In one embodiment, n is independently 3.

In one embodiment, R₁ is independently hydrogen.
In one embodiment, R₁ is independently methyl.

In one embodiment, the α-carbon is in the (R)-configuration.
In one embodiment, the α-carbon is in the (S)-configuration.

Each and every compatible combination of the embodiments described above is explicitly disclosed herein, as if each and every combination was individually and explicitly recited.

Compounds of Formula (2) are similar to compound of Formula (1), except that the groups R₂ and R₃ of Formula (1) together form alkylene chain (e.g., -(CH₂)ₙ-) and, together with the atoms to which they are attached, form a five-, six-, or seven-member lactone ring.

For compounds of Formula (2), the α-carbon may independently be in the D-amino acid configuration (i.e., (R)-configuration) or the L-amino acid configuration (i.e., (S)-configuration. Both enantiomers, and (e.g., racemic) mixtures thereof, are approximately equipotent in biological terms (see below). The chiral center of the lactone is such that there may be no significant energy barriers in assuming either of the active configurations. Thus, a racemic mixture of the lactone may also be useful.

Homoserine (Hse) may be cyclized to form a five-member lactone ring (a γ-lactone ring) referred to as homoserine lactone (Hsl). Consequently, compounds of Formula (2), wherein n is 1, are referred to herein as D-Hsl, L-Hsl, and DL-Hsl analogs.

Examples of some preferred compounds include the following:

| | | |
|---|---|---|
| 1 | (1R,2S,5R)-2-isopropyl-5-methyl-cyclohexanecarboxylic acid ((S)-2-oxo-tetrahydro-furan-3-yl)-amide | 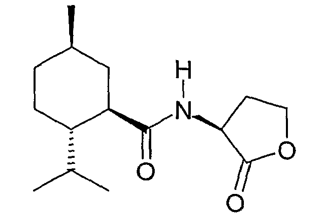 |
| 2 | (1R,2S,5R)-2-isopropyl-5-methyl-cyclohexanecarboxylic acid ((R)-2-oxo-tetrahydro-furan-3-yl)-amide | 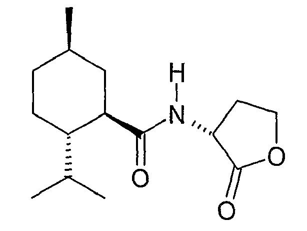 |
| 3 | (1R,2S,5R)-2-isopropyl-5-methyl-cyclohexanecarboxylic acid (2-oxo-tetrahydro-furan-3-yl)-amide | 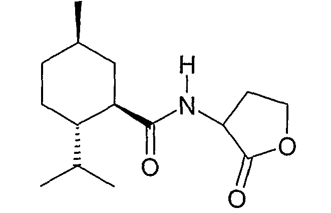 |

Additional examples of preferred compounds include the following:

| | |
|---|---|
| 1 | (1R,2S,5R)-2-isopropyl-5-methyl-cyclohexanecarboxylic acid methyl-((S)-2-oxo-tetrahydro-pyran-3-yl)-amide |
| 2 | (1R,2S,5R)-2-isopropyl-5-methyl-cyclohexanecarboxylic acid methyl-((R)-2-oxo-tetrahydro-pyran-3-yl)-amide |
| 3 | (1R,2S,5R)-2-isopropyl-5-methyl-cyclohexanecarboxylic acid methyl-(2-oxo-tetrahydro-pyran-3-yl)-amide |

Additional examples of preferred compounds include the following:

| | |
|---|---|
| 1 | (1R,2S,5R)-2-isopropyl-5-methyl-cyclohexanecarboxylic acid ((S)-2-oxo-tetrahydro-pyran-3-yl)-amide |
| 2 | (1R,2S,5R)-2-isopropyl-5-methyl-cyclohexanecarboxylic acid ((R)-2-oxo-tetrahydro-pyran-3-yl)-amide |
| 3 | (1R,2S,5R)-2-isopropyl-5-methyl-cyclohexanecarboxylic acid (2-oxo-tetrahydro-pyran-3-yl)-amide |
| 4 | (1R,2S,5R)-2-isopropyl-5-methyl-cyclohexanecarboxylic acid methyl-((S)-2-oxo-tetrahydro-pyran-3-yl)-amide |
| 5 | (1R,2S,5R)-2-isopropyl-5-methyl-cyclohexanecarboxylic acid methyl-((R)-2-oxo-tetrahydro-pyran-3-yl)-amide |
| 6 | (1R,2S,5R)-2-isopropyl-5-methyl-cyclohexanecarboxylic acid methyl-(2-oxo-tetrahydro-pyran-3-yl)-amide |

Additional examples of preferred compounds include the following:

| | |
|---|---|
| 1 | (1R,2S,5R)-2-isopropyl-5-methyl-cyclohexanecarboxylic acid ((S)-2-oxo-tetrahydro-oxepan-3-yl)-amide |
| 2 | (1R,2S,5R)-2-isopropyl-5-methyl-cyclohexanecarboxylic acid ((R)-2-oxo-tetrahydro-oxepan-3-yl)-amide |
| 3 | (1R,2S,5R)-2-isopropyl-5-methyl-cyclohexanecarboxylic acid (2-oxo-tetrahydro-oxepan-3-yl)-amide |
| 4 | (1R,2S,5R)-2-isopropyl-5-methyl-cyclohexanecarboxylic acid methyl-((S)-2-oxo-tetrahydro-oxepan-3-yl)-amide |
| 5 | (1R,2S,5R)-2-isopropyl-5-methyl-cyclohexanecarboxylic acid methyl-((R)-2-oxo-tetrahydro-oxepan-3-yl)-amide |
| 6 | (1R,2S,5R)-2-isopropyl-5-methyl-cyclohexanecarboxylic acid methyl-(2-oxo-tetrahydro-oxepan-3-yl)-amide |

In one embodiment, the compound is in substantially purified form and/or in a form substantially free from contaminants.

In one embodiment, the substantially purified form is at least 50% by weight, e.g., at least 60% by weight, e.g., at least 70% by weight, e.g., at least 80% by weight, e.g., at least 90% by weight, e.g., at least 95% by weight, e.g., at least 97% by weight, e.g., at least 98% by weight, e.g., at least 99% by weight.

Unless specified, the substantially purified form refers to the compound in any stereoisomeric or enantiomeric form. For example, in one embodiment, the substantially purified form refers to a mixture of stereoisomers, i.e., purified with respect to other compounds. In one embodiment, the substantially purified form refers to one stereoisomer, e.g., optically pure stereoisomer. In one embodiment, the substantially purified form refers to a mixture of enantiomers. In one embodiment, the substantially purified form refers to a equimolar mixture of enantiomers (i.e., a racemic mixture, a racemate). In one embodiment, the substantially purified form refers to one enantiomer, e.g., optically pure enantiomer.

In one embodiment, the contaminants represent no more than 50% by weight, e.g., no more than 40% by weight, e.g., no more than 30% by weight, e.g., no more than 20% by weight, e.g., no more than 10% by weight, e.g., no more than 5% by weight, e.g., no more than 3% by weight, e.g., no more than 2% by weight, e.g., no more than 1% by weight.

Unless specified, the contaminants refer to other compounds, that is, other than stereoisomers or enantiomers. In one embodiment, the contaminants refer to other compounds and other stereoisomers. In one embodiment, the contaminants refer to other compounds and the other enantiomer.

In one embodiment, the substantially purified form is at least 60% optically pure (i.e., 60% of the compound, on a molar basis, is the desired stereoisomer or enantiomer, and 40% is the undesired stereoisomer or enantiomer), e.g., at least 70% optically pure, e.g., at least 80% optically pure, e.g., at least 90% optically pure, e.g., at least 95% optically pure, e.g., at least 97% optically pure, e.g., at least 98% optically pure, e.g., at least 99% optically pure.

Preferred among these NACE compounds are "long-acting" NACE compounds that, when applied to the skin or mucous membranes, produce refreshing, soothing, and cooling sensations without skin irritation, with minimal eye irritation, and with a duration of action on skin that lasts more than about 1 hour when used at 40 mM or less, and a duration of action on the linings of the oral cavity and upper respiratory tract that last for more than about 30 minutes. (The concentration of 40 mM corresponds to 1% wt/vol, or 10 mg/100ml, for a compound with a molecular weight of 250 Daltons.)

The long-acting NACE compounds are distinguished from other *N*-alkylcarbonyl-amino acid esters (e.g., WS-5 and WS-31; see Table 2) and *N*-alkyl substituted carboxamides (e.g., WS-3, WS-12, WS-23; see Table 5) (WS-23 is *N*-2,3-trimethyl-2-isopropylbutanamide), which are known to have cooling properties and the two (WS-3 and WS-23) that are commercially used in comestibles, confectionery, and toiletries.

As shown in the examples, WS-3, WS-5, WS-12, WS-23 and WS-31, have a short duration of action (less than 1 hour) at 40 mM or slow onset (more than 5 minutes). Also, some of these compounds do not achieve significant cooling but rather produce skin sensations of tingling, burning, and irritation, effects that are similar to those observed with (-)-menthol, a compound with multimodal actions of sensory processes. Note that (-)-menthol is one of eight stereoisomers of menthol; it has the strongest cooling action and is the one used in commerce.

By contrast, the preferred long-acting NACE compounds deliver a perfect cooling sensation, with rapid onset, long duration of action, and minimal skin or eye irritation that has not been previously recognized.

Furthermore, addition of a methyl group to *N*-p-menthanecarbonyl glycine ethyl ester (WS-5) on either the nitrogen atom or the α-amino carbon increases, by at least 50%, the duration of action.

Furthermore, the potency, duration, and selectivity (absence of irritation) of action are increased for the alanine derivative with the α-carbon in the D-configuration.

Additionally, cyclization of the α-carbon of the amino acid to the alkyl moiety of the ester in order to form a 5-membered γ-lactone ring results in compounds with the desired bioactivity.

Due to their prolonged activity, the compounds, compositions, and articles may be used therapeutically, for example, to reduce discomforts associated with pathophysiological manifestations of injury and inflammation on cutaneous and upper alimentary and respiratory surfaces of the body.

These compounds may be used on skin and in the oral cavity to counteract irritation, itch and pain in therapeutic situations where prolonged relief of sensory discomfort is desired such as for intense pruritic eczema and for severe, sustained, coughing and wheezing.

These compounds inhibit the perception of itch, pain, and discomfort from the skin and the mucous membranes of the oral cavity and upper respiratory tract, and so can be used in the inhibition of sensory disorders in these tissues.

These compounds (for example, when formulated in a lozenge or a liquid at unit doses of up to 10 mg) have rapid onset of less than 1 minute, soothe the throat, and have potent action anti-tussive action exceeding several hours, with no irritation to the mouth or airways.

These compounds are without odor, smarting, or burning sensations on the facial skin or in the mouth and

The compounds are useful by themselves and/or in compositions further comprising a delivery vehicle, such as a delivery vehicle for delivering the compound to skin. In one embodiment, the compound is carried on a towelette adapted for, or of sufficient construction for, the delivery of a dermatologically effective amount of the compound to the skin.

The prolongation of action permits use of the long-acting NACE embodiments in therapeutic situations where discomfort of skin or mucous membranes is present for at least one day, for example, after photodynamic or laser surgery of the facial skin, dermatitis of the facial skin, or even after severe insect bites.

Currently, there are no topical analgesic medications approved in this category for therapeutic relief of skin discomfort, although the demand exists for such substances. The potent D-Ala and Hsl derivatives, among others, with selective cooling and refreshing actions increase the scope of agents that may be used in the oral cavity and upper respiratory tract and may be incorporated into therapeutics such as anti-tussive and antiasthmatic formulations.

The specific structural features of the molecules that confer the desired properties of increased potency and the presence of refreshing cooling without irritation were unexpected and surprising and not known in the prior art.

The term "treatment," as used herein in the context of treating a condition, pertains generally to treatment and therapy in which some desired therapeutic effect is achieved, for example, the inhibition of the progress of the condition, and includes a reduction in the rate of progress, a halt in the rate of progress, alleviation of symptoms of the condition, amelioration of the condition, and cure of the condition. Treatment as a prophylactic measure (i.e., prophylaxis) is also included. For example, use with patients who have not yet developed the condition, but who are at risk of developing the condition, is encompassed by the term "treatment."

The term "effective amount," as used herein, pertains to that amount of an active compound, or a material, composition or dosage form comprising an active compound, which is effective for producing some desired therapeutic effect, commensurate with a reasonable benefit/risk ratio, when administered in accordance with a desired treatment regimen.

### Pharmacology and Mechanisms of Action of N-Alkylcarbonyl and N-Alkyl-N-alkylcarbonyl- Amino Acid Esters and Lactones

Noxious stimuli from the skin are thought to be transmitted by unmyelinated C fibers and thinly myelinated Aδ fibers. Functionally, these fibers are also called polymodal and may contain in one group neuropeptides such as calcitonin-gene related peptide and substance P, or in another group contain phosphatases and binding sites for isolectin B4. These sensory fibers also contain various receptors, including the transient potential receptor (TRP) potential family of receptors that code for thermosensation and pain. The afferent signals for initiation of cough originate from detectors on the surface of trachea and larynx and are transmitted via the laryngeal branch of the vagus. These Aδ-fibers are from neurons within the nodose ganglion and contain rapidly adapting receptors. Sensitivity to cough stimuli is separately enhanced via C-fibers by protons, inflammation, and vanilloid receptor TRPV1 agonists such as capsaicin, but the precise detector and transducer circuitry of the C-fibers has not been elucidated (see, e.g., Canning et al. J. Physiol. 557: 643-548, 2004).

There are many conditions that produce sensory discomforts on the skin and in the mucous membranes of the mouth and upper airways, discomforts which are ameliorated by cooling (*vide infra*). Cooling of the facial skin and mucous membranes is detected by a subset of primary sensory afferents that have receptors on nerve endings. These sensory fibers exhibit a rhythmic, ongoing discharge at neutral temperatures that increases in response to skin temperature cooling (step reductions from 33 to 23°C) and are suppressed by warming. The dynamic information is propagated along axons in spike trains, at about 20 to 40 impulses/sec, to central neurons, leading in humans to cooling sensations. This type of sensation is mimicked by facial exposure to air or water temperatures of 15 to 22°C. The primary afferents from facial skin terminate in the superficial layer of the caudal trigeminal nucleus where they represent over 95% of the thermoceptive input (see, e.g., Hutchinson et al., J. Neurophysiol., 77:3252-3266, 1997). The cooling signals from the nasopharynx and the oropharynx are transmitted via the glossopharyngeal nerve.

Temperature detection inputs from the face and lips are especially important for modulating behavior as this surface is densely innervated, as can be seen in diagrams of the sensory homunculus in textbooks of psychology. This fact is readily experienced as we notice temperature changes easily from sensations on our face but less so from other parts of our body. The three branches of the trigeminal nerve - ophthalmic, maxillary, and mandibular - send afferent thermosensitive information from the cutaneous surfaces of the face, head, scalp, lips, oral membranes, and dorsal surface of the tongue to surface nuclei of the brainstem. These thermoceptive units on the trigeminal nerve respond dynamically to drops in surface temperatures and are inhibited by warmth. Thermosensation from the face is dominated by these cold receptor signals, as heat detection is not tonically active.

Coolness signals detected from the oral cavity are more complex, because the precise identification of the signal may be confounded by variables such as the secretion of saliva and gustatory signals mediated by the facial and glossopharyngeal nerves that are distinct from the trigeminal input. Sensory signals from the oral cavity, trachea, and larynx originate from detectors on nerve endings in branches of the trigeminal nerve, the lingual nerve, the hypoglossal nerve, and the superior laryngeal nerves of the vagus.

The precise mechanisms underlying the benefits of refreshing cooling on sensory discomfort are not clearly understood, although such benefits are a common experience. Sensations can be "confusing" when a chemical affects more than one sensory modality. This is especially true for (-)-menthol (also known as L-menthol, (1R)-menthol, and (1R,2S,5R)-menthol). (-)-Menthol is widely used as a cooling agent but it has multimodal action on sensory processes. For example, in the upper airways and oral cavity, (-)-menthol can elicit somatosensation (cooling, irritation, tingling), olfaction (minty), and gustation (bitter). As a counter-irritant, (-)-menthol can reduce irritation of oral and pharyngeal membranes (e.g., strong mints or toothpastes) and have analgesic actions on muscle (e.g., BenGay® ointment). The multimodalities may further mix to give rise to complex perceptions of irritation (burning, prickling, stinging), especially around the eyes, of thermal effects (cooling, warming) and of tactile effects (buzzing, tingling, tickling, numbing). In the nose and oral cavity, the predominant mode of detecting (-)-menthol is olfactory (see, e.g., Nagata et al., J. Exptl. Psychol., 31: 101-109, 2005).

It is not clear if the anti-nociceptive actions of (-)-menthol are directly mediated on peripheral structures such as nociceptors, or indirectly mediated via activation of cool/cold receptors on "cool" nerve endings. In the latter case, suppression of nociception is via central "gating" of the incoming noxious and irritative signals. The peripheral (-)-menthol coolness receptor is thought to be a protein called TRP-M8. However, it has been found that the potency of compounds that activate TRP-M8 is not correlated to cooling actions (see, e.g., A.K.Vogt-Eisele, D. Bura, H. Hatt, and E.T.Wei. N-Alkylcarboxamide Cooling Agents: Activities on Skin and Cells with TRPM8 and TRPA1 Receptors. 3rd Annual Workshop on the Study of Itch, September 25 to 27, 2005 in Heidelberg, Germany. Acta Dermato-Venereological 85: pg.468, 2005). Furthermore, TRP-M8 is activated by mustard oil, an agent that produces the pungent sensations of *wasabi.* Thus, the compounds of the present invention should not be viewed as solely acting via TRP-M8 receptors.

The multimodal action of (-)-menthol and related agents on sensory processes are utilized in compositions for food, confectionery, flavors, chewing gum, lipsticks, and other comestibles (items put in the mouth), beverages, tobacco products, toiletries, over-the-counter pharmaceutical compositions for treatment of nasal and airway symptoms, for gastrointestinal tract distress, and as a counter-irritant for alleviating discomforts of skin, muscle, and membranes of the oral cavity and throat. Although menthol preparations, such as confectionery, have alerting effects on the central nervous system, menthol compositions cannot be applied to facial skin in effective concentrations to arouse because it causes eye irritation (stinging, smarting, tearing, and pain).

Thus, identification of an agent that does not irritate the eye surfaces, but which can be applied to facial skin to arouse would have utility. Furthermore, such an agent can be applied to skin around the eyes to refresh and to reduce skin irritation, itch and pain.

Cosmetic menthol-like cooling compounds in commercial use for topical applications to skin are principally menthyl lactate (Frescolat® ML), menthone glycerin acetal (Frescolat® MGA), and menthoxypropanediol (Cooling Agent 10). These agents do not act on the facial skin for longer than 30 minutes. Two other agents, WS-3 and WS-23, are mainly used in confectionery, chewing gum, and toiletries (mouthwashes, after-shave lotions, shampoos, deodorants, toothpastes). The recent information on cooling agents used for skin applications was reviewed by M.B. Erman (Cooling agents and skin care applications. Cosmetics & Toiletries 120: 105-118, May 2005; Progress in physiological cooling agents. Perfumer & Flavorist 29: 34-50, 2004) and by P. Jacobs and W. Johncock (Some like it cool. Parfumerie und Kosmetik 80: 26-31, 1999).

None of the agents in this group satisfy criteria for therapeutic use on the skin because (a) pharmaceutical formulations generally require a single active ingredient and (b) none of the current cooling agents has sufficient duration of action to be therapeutically valuable.

In order to treat skin discomfort, a compound must act for at least one hour and preferably longer, otherwise the patient would have to repeatedly apply the drug to obtain relief. For an anti-irritant or anti-tussive action in the airways, the ideal agent should have rapid onset of action, soothing effects, and the ability to relieve discomfort for an extended duration, for example, for several hours.

### Non-Technical Description of Inventive Concept

Using recording electrodes, cool and hot signals entering the brain (see Hutchinson et al., *vide supra*) were converted to audio signals. The "cool" neurons generate a "pitter-patter" sound, like raindrops falling on a rooftop. These neurons are tonically active at room temperature. Further cooling, for example, with an ice-cube brought near the receptive surface, increases the sound and frequency of the "pit-pat" to that of a strong shower, but never that of a downpour or a torrent of discharges. By contrast, the "heat/pain" neurons are silent until a heat source brings the skin temperature near 43.3°C. Then these neurons discharge in synchrony with a roar, like the sound of high surf or tide coming onto a beach. The pit-pat and roar of cool and hot neurons are modality specific and not activated by pressure or touch. It is believed that the long-acting NACE compounds described herein set the pitter-patter transmission of cool neurons so that the brain perceives the ambient temperature at about 15 to 18°C. Activation of these neurons is like turning on a robust air-conditioner within a hot environment. This sensory band in normal individuals is felt as alerting, refreshing, and cool. This is referred to herein as the "perfect cool." The presence of the NACE compounds and the perfect cool, in pathological conditions, gates the passage of noxious signals into the spinal cord and/or brain. Hence, a soothing anti-nociceptive (anti-irritant, anti-pruritic and anti-tussive) effect is achieved with therapeutic benefit.

The inventor has identified molecules with potent and prolonged activation of the perfect cool. These molecules are qualitatively and quantitatively unlike (-)-menthol and WS-3 which act for less than 20 minutes. In certain cases of itch and cough, it was also observed that the NACE compounds exerts prolonged anti-nociceptive activity when the sensations of the perfect cool no longer reach conscious perception, and that repeated applications of the NACE compounds can silence and extinguish nociception. These results suggest that the perfect cool may further modulate and attenuate the plasticity of the nociceptive process.

The long-acting NACE compounds are active at single doses of 1 to 10 mg and at concentrations of 10 mg/mL or less when applied topically to the surfaces of the body. By topical, it is meant that the application is onto surfaces of the body in contact with air, which includes the facial skin, the eyelids, the lips, the upper and lower respiratory tract, and the entrance and exit of the gastrointestinal tract, namely, the oral cavity and the anorectum.

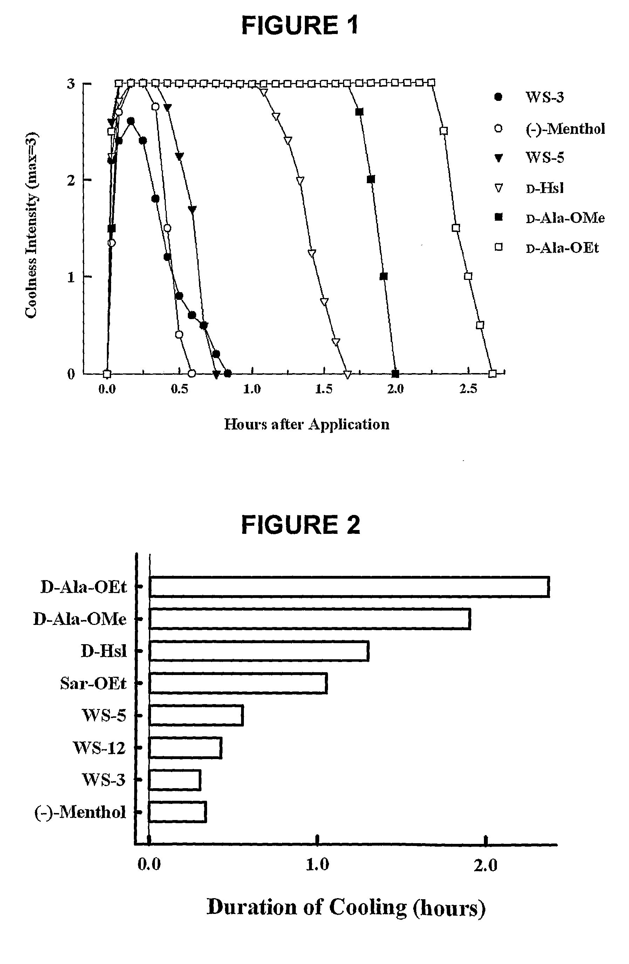

The long-acting NACE compounds also have a rapid onset of action (from about 0.5 to about 3 minutes) relative to other compounds (see, e.g., Figures 1 and 2 and Examples). The onset and offset of action of these compounds was first revealed by testing on the facial skin of subjects and then subsequently by applying them into the oral cavity.

### Bioassays of N-Alkylcarbonyl-, N-Alkyl-N-Alkylcarbonyl- Amino Acid Esters and Lactones

Psychic events such as refreshment, soothing, cooling, irritation, itch, and pain cannot be verbalized by animals (animals cannot say "it feels cold", "ouch", or that "it itches"). Hence, the sensory effects of chemicals in animals must be indirectly inferred. Receptor assays, based on cells transfected with the genes for proteins associated with thermosensation (e.g., TRP-M8, TRP-A1, TRP-V1) may be used as a model of sensory processes. The receptor assays yield quantitative data. However, these assays give no information on onset and offset of action, or on the quality of human sensations evoked by the chemicals. Furthermore, potency as measured by the median effective concentrations (EC₅₀) in the receptor assays may not be correlated to anti-nociceptive or cooling actions. Thus, the best information on the pharmacological properties of chemicals is derived from direct tests on humans.

Watson et al. (US Patent No. 4,178,459) tested the properties of *N*-substituted p-menthane carboxamides on volunteers by putting filter paper (1x1 cm), impregnated with a known amount of compound, onto the dorsal surface of the tongue of the test subject. After 30 seconds, the subject was required to report presence or absence of a cooling effect. These data were reported as "Threshold, µg" and refer to the threshold amount of the test substance that produces cooling sensations upon application onto the tongue of a panel of human volunteers. The average threshold of (-)-menthol for 6 subjects was 0.25 µg, but there was a 1 00-fold variation in individual sensitivity. As noted above, coolness signals detected from the dorsal surface of the tongue may be confounded by gustatory, olfactory, and other variables, as well as by dilution from saliva.

It has been found that, if the goal is to find a drug useful for topical application, the refreshing cooling and sensory properties of a long-acting NACE compound are best tested first by suspending or dissolving a test substance in an ointment (usually Aquaphor®, which is 41 % petrolatum, and the rest mineral oil, ceresin and lanolin alcohol) and singly applying the ointment (40 to 70 mg) onto the skin surface using a plastic stick. A reliable place for topical application is the skin above the upper lip (above the vermilion border of the lips), on the philtrum, lateral to the philtrum until the nasolabial folds, and on the lower nostrils (subnasale). This part of the face is known to be densely innervated with cold receptors, second only to the surfaces of the eyeball and anogenitalia. A second location is on or below the malar eminence (cheekbone). The skin above the cheekbone is thicker than above the lips, and therefore has a higher threshold for activation. At both loci, tingling, cool and cold sensations in the skin may be experienced and rated for time of onset and intensity.

The intensity of the subjective skin sensation is rated as 0, 1, 2 or 3 with: 0 as no change; 1 as slight coolness, cold, or tingling; 2 as clear-cut signal of coolness, cold, or tingling; and 3 as robust cooling or cold. The intervals for recording sensations are 5 to 10 minutes, until two successive zeroes are obtained. The results (shown in Figures 1 and 2) are averaged values of 4 to 6 separate trials in the same individual. The data are plotted using SigmaPlot® (Systat Software, Point Richmond CA) and a smoothing function with a negative exponential was used for analysis and statistical fit of the results. Confirmatory trials of cooling action of the long-acting NACE compounds were obtained in 2 to 4 individuals but not quantified for some because of the large number of chemicals that were evaluated.

The onset of drug action is taken as the time to reach 2 units of coolness intensity, and offset of drug action is the time when coolness intensity drops below 2, after previously surpassing 2 units. The duration of cooling action is defined as the offset time minus the onset time. An inactive compound is defined as one that does not exceed 2 units of cooling for 5 minutes after application. The quality of the sensation is noted, for example, as pure refreshing coolness, or if the sensation is accompanied by irritation (stinging or burning). The quality of the sensation is not rated for intensity.

The ointment is also applied to the periorbital skin (upper and lower eyelids and on skin adjacent to the lateral canthus) for tests of irritancy near the eyes, and the subject is asked if irritation is present or absent. The intensity of the eye sensation is not rated.

The sensory information described above is not obtainable in a receptor assay. For a topical agent, the most reliable results are therefore first derived from direct tests on the skin. The next target for testing is the receptors in the oral cavity and the upper airways. Taste thresholds are difficult to quantify on the tongue because of the dynamic fluid conditions in the oral cavity and the presence of taste and adaptive factors that affect thermosensation on the tongue. The oral cavity tolerates extremes of hot and cold temperatures that are not acceptable on the skin. For example, hot beverages (such as coffee) are tolerated at temperatures in the mouth that are considered painful and scalding when spilled on the skin. Also, ice-cold drinks may be refreshing in the mouth, but an ice-cube on the skin quickly becomes unpleasant.

It has been found that the most effective method for testing a compound for effects in the oral cavity is to take a 1 to 5 mg sample and place it on the dorsal surface of the tongue, 2/3 posterior from the tip and in the midline. The subject is instructed to close and hold the substance in the mouth for at least 10 seconds and not to swallow. The description of sensations is then recorded at 5 minute intervals.

### Qualitative Aspects of Coo/ and Cold Intensity

The static and dynamic temperatures of the skin give rise to sensations that are qualitatively distinct. The normal skin temperature is 32 to 34°C and when water is applied to the skin, it is called: tepid at 27 to 32°C; cool between 18 to 27°C, cold at 13 to 18°C, and very cold below 13°C. A critical range of room temperatures for coolness and cold is at 18 to 22°C. For example, a sedentary individual, dressed lightly, will frequently want to turn up the thermostat when the room temperature drops one or two degrees below 20°C (68°F). In gaming establishments, air temperatures are deliberately kept at 18 to 20°C in order to arouse, increase vigilance, and activate gambling activities. In the outdoors, breathing cool air at 15 to 21 °C is refreshing, invigorating, and alerting, and the emotional response may be positive and joyful. At ambient or surface skin temperatures of 15°C (55°F) or below, however, the cold sensation become painful and aversive and is accompanied by affect; that is, the person considers these cold sensations to be unpleasant, seeks to escape the environment, and may become angry, hostile, or irritable if escape is not possible.

The long-acting NACE compounds described herein are useful as a topical agent for the relief of skin discomfort, and mimic the effects of running cold water on injured skin. The "nominal" ambient skin surface temperature to mimic with a cooling agent is in the range of 15 to 22°C. The effect can also be simulated by putting a towel wet with water at room temperature onto the face. The coolness of a wet towel will rapidly dissipate, an effect called adaptation, even when the cooling stimulus is still there. On the other hand, for a chemical agent applied to the facial skin, the stimulus is more constantly present. The exact physiological sensation to replicate with the inventive compounds is that of refreshing, soothing coolness, with minimal or no sensations of irritation or sting, and the absence of excessive cold.

As shown in the Examples, the preferred long-acting NACE compounds, tested at 40 mM, produce cooling sensations on the facial skin, have a rapid onset of action (less than 5 minutes) and slow offset (more than 1 hour). By contrast, various structurally similar compounds were either inactive or had a short duration of action, as shown by comparative data in Figures 1 and 2 and in the Examples.

The long durations of action of some preferred NACE compounds was unexpected and surprising. Some of the NACE compounds, especially D-amino acid derivative of alanine and the Hsl analogs, were found to have cooling actions for several hours, an astonishing and unexpected effect.

On the tongue of normal subjects, the effects of a NACE compound may elicit an initial "tickle" of 1 to 3 seconds, then the onset of robust and refreshing cooling could be detected. Usually, at a dose of 1 to 2 mg of a NACE compound, such as the D-Ala-OMe or D-Ala-OEt analogs, the cooling sensations lasted for about 30 minutes. Surprisingly, in subjects with cough or irritative stimuli originating from the throat, there was rapid (within 1 minute) attenuation of sensory discomfort and the cessation of coughing. Also surprisingly and unexpectedly, the duration of the anti-tussive effect lasted for at least several hours and occurred when the cooling sensations were only minimally detected, if at all. This soothing effect on the oropharynx and throat was especially beneficial to two asthmatic subjects who had frequent cough, chest tightness, and wheezing.

### Structure-Activity Relationships of Compounds Producing the Perfect Cool.

The idea of a chemical agent, able to maintain a sustained refreshing, soothing, and cooling effect with minimal irritation or sting, implies a special relationship of the molecule with the receptor on the nerve endings. Potency and selectivity of chemical agonists at receptors are basic concepts of drug action. A strong candidate for the cool receptor is TRP-M8 because it is present in sensory neurons and responds to temperature decrements and to cooling agents such as icilin and menthol. However, *N*-p-methoxyphenyl-p-menthane-carboxamide (WS-12) is more potent at TRP-M8 than WS-5 (Gly Et Ester), yet WS-12 has less cooling action than WS-5 and a much slower onset of action. This observation shows that potency at the TRP-M8 receptor is not a key determinant of the perfect cool (see also Vogt-Eisele et al., *vide supra*). Surprisingly, as shown in Table 5, there is also no correlation, for eleven compounds, between potency for cooling action on the tongue and the duration of cooling on facial skin.

It is possible that long-acting NACE compound act on more than one receptor type to confer the perfect cool. A number of receptors linked to noxious stimuli are present on sensory neurons (TRPA1, TRP-vanilloid receptors, peptide receptors such as those for substance P and bradykinin, histamine receptors, and K⁺ channels). Some, for example TRP-V1, have binding sites of activation (on intracellular transmembrane loops 2 to 3 of the receptor) that are similar to the active binding site of TRP-M8. In the absence of precise knowledge of the noxious receptors that cause warmth, heat, irritation, itch, and pain, the question of the receptor selectivity of long-acting NACE compound cannot be resolved at this time.

The access and residence of the agonist molecule at the receptor sites in an important factor for drug action. A key pharmacokinetic determinant of drug distribution and delivery on the skin is the octanol/water partition coefficient. Thus, the logarithm of the octanol/water partition coefficient (log P) in the range of 2.0 to 4.0 is considered ideal for activation of cool receptors in the skin. However, in the Examples, the log P of Sar Et Ester, a long-acting NACE compound, is the same as the non-NACE analogs, such as WS-11, without equivalent "perfect cooling" by WS-11. Sar Et Ester has a duration of action that is five times greater than that of WS-11, but on the tongue threshold test, it is 2.7 times less potent than WS-11.

An analysis of the structure-activity relationships from the present studies indicates that the presence of a single ester group, preferably a methyl or ethyl ester, on *N*-substituted-p-menthane-carboxamides, confers desirable biological activity, but by itself is not sufficient for prolonged cooling (>1 hour). The presence of additional polar entities on the amino acid ester, for example, hydroxyl, alkoxy, and/or diesters groups, such as in Ser or Glu(OMe) derivatives, decreases potency. Substitution of a methyl group for the hydrogen on the amino function, to give *N*-methyl-glycine (Sar Et Ester) prolongs duration of activity. An *N*-alkyl substitution has an effect on the cis-trans ratio of the amide bond, lowers the relative energy of the cis isomer, and may also interfere with enzymes (e.g., amidases) that break down the amide bond. In addition, the data show that activity is prolonged if the α-carbon of the glycine bears a one or two carbon alkyl substituent (2-amino-*n*-proprionic acid or 2-amino-*n*-butyric acid), for example, to give an alanyl ester derivative. Furthermore, if the steric orientation at the α-carbon atom is in the D-configuration instead of the L-configuration for alanine or if the α-carbon atom is cyclized with the alkyl ester to form a lactone ring, then potency, duration, and selectivity of preferred sensory effects are enhanced.

The benefits of α-carbon-alkylation in the D-configuration, and the cyclization of the α-carbon to the alkylester to form a lactone, as important determinants for prolonged refreshing cooling (with the absence of irritation) are new observations.

It is also recognized that although the preferred cycloalkyl group is p-menthane, where the cycloalkyl is 2-isopropyl-5-methylcyclohexyl, other rings structures such as alkyl-substituted cyclopentyl, cycloheptyl and cyclooctyl, or alkyl-substituted bicycloheptyl and bicyclooctyl are also efficacious. Furthermore, the cycloalkyl moiety may be replaced by a branched chain aliphatic group, such as in WS-23, and the affinity for the cooling actions is retained.

This structure-activity relationship gives rise to the following class of compounds with the perfect cool, specifically, compounds selected from compound of Formula (3), and salts and solvates thereof: 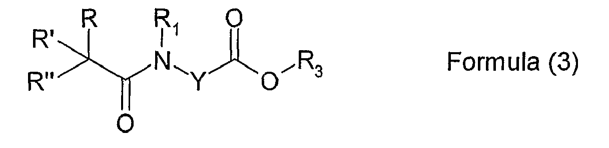 wherein:
each of R and R' is independently C₁ to C₇ alkyl; and
R" is independently hydrogen or C₁ to C₅ alkyl;
with the provisos that:
(1) R, R', and R" together provide a total of at least 5 carbon atoms, and preferably a total of 7 to 13 carbon atoms; and
(2) if R" is hydrogen, then: R must contain at least 2 carbon atoms and R' must contain at least 3 carbon atoms, and at least one of R and R' must be a branched alkyl group (preferably with branching at the α- or β-position relative to the C-CO-N group); and
(3) if R" is hydrogen, then: it is preferred that at least one of R and R" is isopropyl or sec-butyl;
or:
R and R', taken together with the carbon to which they are attached, form an alkyl-substituted cycloalkyl group selected from: cyclopentyl, cyclohexyl, cycloheptyl, cyclooctyl, [3.1.1]bicyloheptane, [2.2.1]bicyloheptane, and [2.2.2]bicyclooctane, and optionally has ethylenic unsaturation (preferably the menthyl group found in (-)-menthol); and
R" is hydrogen or linear or branched C₁ to C₅ alkyl;
with the provisos that:
(i) the alkyl-substituted cycloalkyl ring contains a total of 7 to 14 carbon atoms (and preferably a total of 8 to 12 carbon atoms); and
(ii) the alkyl-substituted cycloalkyl ring has from 1 to 3 linear or branched C₁ to C₅ alkyl substituents; and
(iii) if R" is hydrogen, then: a linear or branched C₁ to C₅ alkyl substituent is present at the 2- or 3- position of the cycloalkyl ring (preferably at the 2-position) (preferably said alkyl substituent is branched at the α- or β-position relative to the ring) (preferably said alkyl substituent is isopropyl or sec-butyl);
and wherein:
R₁ is hydrogen or methyl;
Y is a divalent alkylidene moiety selected from -CHR₂-, -CH₂CHR₂-, and -CHR₂CH₂-, wherein R₂ is hydrogen or C₁ to C₂ alkyl; and
R₃ is linear or branched C₁ to C₄ alkyl;
with the provisos that:
(a) additionally, R₂ and R₃, together with the atoms to which they are attached, may form a 5-, 6-, or 7-membered lactone ring; and
(b) additionally, R₁ and R₃, together with the atoms to which they are attached, may form a saturated 5-, 6-, or 7-membered 3'-oxo-1',4'-azoxa ring; and
(c) additionally, R₁ and R₂, together with the atoms to which they are attached, may be linked to form a 5-, 6-, or 7- saturated nitrogen heterocycle ring having an alkoxycarbonyl substituent at the 2' or 3' position and optionally substituted with one or more C₁ to C₂ alkyl groups.

These compounds (i.e., selected from compounds of Formula (3) and salts and solvates thereof) are also useful in the methods described herein, and in particular, in methods of preventing coughing and airborne transmission of an infectious microorganism, and in methods of reducing host dissemination of an infectious microorganism.

### Uses of Long-Acting NACE Compounds on the Face and Other Surfaces

In a preferred use, one or more long-acting NACE compounds is topically applied to therapeutically relieve the irritation, itch, and/or pain of inflamed skin and/or of inflamed tissues in the oral cavity and/or upper respiratory tract.

Other contemplated uses include long-term refreshment of the facial skin and to increase alertness and vigilance.

Contemplated non-therapeutic uses include use as ingredients in comestibles (e.g. chewing gum, mouth-washes, anti-gingivitis products, toothpastes), cosmetics, lipsticks, flavors, tobacco additives, confectionery, or toiletries.

By "topically" it is meant application onto surfaces of the body in contact with air, which includes the skin, the eye surface, the lips, the upper (nose) and lower respiratory tracts, and the entrance and exit of the gastrointestinal tract, that is, the oral cavity and the anorectum. Particularly favored sites of application are the surface tissues of the face and head innervated by the trigeminal nerve which includes the skin, scalp and the linings of the orbit, lips, nose and mouth. A second favored site is the nerve endings of the glossopharyngeal nerve in the naso- and oro- pharynx.

By "oral administration" it is meant delivery of the active ingredient as solid, liquid, or aerosol, into the oral cavity, preferably in a delivery vehicle.

For topical uses, a long-acting NACE compound is preferably formulated so as to have fast onset and slow offset.

Preferably, the compound does not sting or irritate when applied on the face near the orbit, and produces more refreshing cool than cold.

Some of the uses may be further categorized as:
Therapeutic: A long-acting NACE compound may, for example, be used as a local analgesic on inflamed skin or as an anti-pruritic. It may also be orally administered as an anti-tussive or anti-irritant for the treatment of asthma.
Anti-irritant: A long-acting NACE compound may be incorporated into a skin care product that contains irritating substances, such as retinoids or α-, or w- fatty acids.
Arousal: In normal, healthy individuals, the long-acting NACE compounds may be used to alert and to refresh, to counteract fatigue, and to relieve the individual from heat exhaustion, nasal and eye irritation, and obstructed breathing discomfort. It may be used to enhance a bright-eyed and alert look because of its refreshing properties. It may be valuable for athletes training in a hot environment, for example, baseball players in Arizona during spring training or soccer players in Spain or South America.
Cleansing: A long-acting NACE may be incorporated into a towelette for removing make-up, especially for mascara around the eyes.
Food and personal care products: A long-acting NACE compound may be incorporated into comestibles (e.g., chewing gum, toothpastes), cosmetics, lipsticks, flavors, confectionery, tobacco, beverages, or toiletries, to provide sensory refreshment.

Therapeutic uses for topical formulations of one or more long-acting NACE compounds are contemplated in a towelette, lotion, cream, ointment, or in oral or inhaled formulations, and include utility for:
(a) alleviation of irritation, itch, and/or pain from various forms of dermatitis (atopic, contact, and irritant),
(b) pain from burned, traumatized, or irritated skin (e.g., laser surgery), and from procedures related to wound debridement,
(c) itch and discomfort from skin infections, insect bites, sunburn, photodynamic treatment of skin (e.g., actinic keratoses, basal cell carcinoma),
(d) pruritus due to xerosis,
(e) mucositis and stomatitis from, for example, apthous ulcers or cancer chemotherapy, cheilitis or itching of the lips from cold sores and gingivitis,
(f) pruritus ani, hemorrhoidal discomfort, pain from anal fissures, pain or itch from anal fistulas, pain from hemorrhoidectomy, perineal inflammation, anogenital skin inflammation, and discomfort due to various local causes such as incontinence, diaper rashes, perineal inflammation,
(g) vulval pruritus and pain (e.g., from candidiasis or idiopathic, such as vulva vestibulitis and vulvodynia), dyspareunia, anogenital infections, including warts and sexually transmitted diseases, viral infections of the skin (especially in immunocompromised patients),
(h) nostril and nasal or upper airway discomfort from breathing obstruction, e.g., rhinitis, asthma, bronchitis, emphysema and chronic obstructive pulmonary diseases, dyspnea, sleep apnea and snoring, and
(i) conjunctivitis, ocular surface irritation, pain from corneal abrasions, and pain from eye surgery.

As sensory processes are also important in hollow viscus, the long-acting NACE compounds may be delivered orally, or inhaled, or encapsulated for systemic and topical delivery to the gastrointestinal tract, the urinary bladder, and the airways. For the gastrointestinal tract, the long-acting NACE compound may be used to relieve heartburn, peptic pain, and the discomforts of irritable bowel and inflammatory bowel diseases. The preferred method of delivery for the lower gastrointestinal tract would be enteric-coated capsules. Alternatively, a long-acting NACE compound, such as the Sar Et Ester analog, which exists as a liquid oil at body temperature, may be extruded onto the gut surface using a controlled release device such as an osmotic minipump. For the urinary bladder surface, an oral formulation of a long-acting NACE may be used to suppress the discomforts of interstitial cystitis and relieve the symptoms of overactive bladder. For the upper airways, long-acting NACE compound, inhaled or delivered as a mist or spray, may mimic (-)-menthol and relieve congestion, relax bronchial smooth muscles, and symptomatically relieve the choking sensations of dyspnea.

### Use of Long-Acting NACE in Anti-Ageing Applications

As lifespan increases, individuals seek therapeutic procedures that allow them to cope with ageing skin. With age, wrinkles, discoloration, and changes in the growth and texture of the skin appear. Various carboxylic acids, for example, hydroxyacids and retinoic acid are topical treatments of these skin conditions. These agents achieve their effects by causing peeling of the upper skin layers. Another method of rejuvenating ageing skin is to resurface the skin with mechanical, chemical, photodynamic or laser (thermal) energy. Resurfacing the skin results in a dermal wound with associated skin discomfort. The long-acting NACE compounds may be used to alleviate the discomforts associated with skin damage caused by anti-ageing procedures and therapies.

With age, the skin is less able to retain proper moisture content. This condition, known as xerosis of the elderly, is manifested as itchy, dry, and fissured skin with scaling and sloughing. In some, xerotic skin is like cracked porcelain. The skin splits because of water loss and, if deep, disrupt capillaries and causes bleeding. Itching leads to scratching and rubbing, activities that exacerbate the pathology and produce a leatherlike condition called lichenification. A provocative factor for xerosis is cold, dry weather, such as in winter or air conditioning. A study of nursing homes found xerosis and eczema was diagnosed in 29% and 37% of the patients, respectively. Therapy includes frequent application of moisturizers and steroid ointments, but will be aided by long-acting NACE compositions delivered in an ointment to relieve skin discomfort. Similarly, disorders of the skin that occur in icthycosis vulgaris and psoriasis may be so treated.

### Use of Long-Acting NACE in Cough, and Airway Irritation and Obstruction

The sensations of stimuli that initiate cough and the physical act of coughing represent the body's response to airway irritation and obstruction. The causes of airway irritation and obstruction are multifactorial and include for acute coughing such conditions as viral or bacterial upper airway infections, post-nasal drip, allergies, inflammation of the airways from air pollutants, pharyngitis, laryngitis, and for chronic coughing such conditions as asthma, chronic obstructive lung disease, gastroesophageal reflux disease, lung cancer, pneumonia, sleep apnea, snoring, pulmonary edema, congestive heart failure, and dyspnea. It has been found that the NACE compounds gate and over-ride signals of airway irritation and obstruction and thus attenuate the sensations of stimuli that initiate discomfort in the throat, for example, from pharyngitis (sore throat) and laryngitis (hoarseness and loss of voice). The desire to cough is blunted and the frequency of coughing is reduced. The sense of airway obstruction, however, is not affected and voluntary coughing can be readily initiated to clear the obstruction.

A descriptor of the actions of the long-acting NACE compound in cough would be as a "cough antagonist" and not as a "cough suppressant" because the ability of the individual to cough is not suppressed. Instead, the NACE compound is soothing the irritative sensations that initiate involuntary coughing. The actions of NACE are qualitatively more intense, penetrating, refreshing, and cooling than cough drops that contain 10 mg of (-)-menthol, the maximum normally present in OTC preparations. The site of NACE compound action is most likely on sensory nerve endings of the glossopharyngeal nerve in the oropharynx. The afferent signals to the brainstem then "gate" or attenuate the irritative signals for cough coming in from the afferents of the laryngeal branch of the vagus. The net effect is that the cough signals are no longer perceived as irritating or sufficient to initiate the cough reflex. Other possible mechanisms of NACE action on the upper respiratory tract are to directly relax bronchial smooth muscles and/or to reduce airway hyper-reactivity or hyper-responsiveness to inflammatory mediators.

When used in the treatment of asthma or chronic obstructive pulmonary diseases, the long-acting NACE compound may be used in combination with a β-adrenoreceptor agonist such as formoterol, an anti-inflammatory glucocorticosteroid such as betamethasone, or a muscarinic receptor antagonist such as ipratropium or tiotropium.

### Use of Long-Acting NACE to Limit Viral Transmission in a Flu-Epidemic

Methods for controlling influenza epidemics are limited to strategies such as vaccines, isolation of infected individuals (quarantine), and the use of neuraminidase inhibitors. Viruses are shed from the infected host via expectoration of large particles, droplets, and inhalable-sized materials. Sneezing is not a characteristic sign of flu, but coughing occurs frequently. A sore throat and cough usually begins 3 days after infection and cough may persist for 3 weeks.

In a quantitative model for estimating risk of infection from respirable pathogens, Nicas et al. (J. Occupational and Environmental Hygiene 2: 143-154) showed that the potential for infection from airborne pathogens is directly correlated to cough frequency. It has been found that a single individual, in an enclosed space for 4.5 hours, can infect 72% of neighbours with flu (M.B. Gregg. The epidemiology of influenza in humans, Ann. N.Y. Acad. Sciences, 353: 45-53, 1980). This sick individual was described as having a "dry, brassy cough."

Use of a potent NACE compound as a cough suppressant to reduce virus dissemination and transmission in an influenza epidemic is a new concept. It may be especially useful to help protect health workers who treat infected individuals. Cough inhibition may be a general method to limit viral infections, for example, as might occur in schools, hospitals, or other areas of crowding. This method of restricting contagion and spread may also be applied to pathogenic respiratory viruses such as respiratory syncytial virus, rhinovirus, and coronaviruses.

### Use in Long-Acting NACE Smoking Cessation Therapy

Mentholated tobacco brands represent approximately 25% of the cigarettes sold in the United States. In some countries, such as the Philippines, it may be as high as 60%. Within the United States, 69% of African-Americans and 22% of Whites smoke mentholated cigarettes. The ethnic disparity in menthol preference is attributed to deliberate marketing of the cigarettes to sub-populations with the message that mentholated cigarettes are: (a) healthy and medicinal, (b) cool, clean, crisp, fresh, and refreshing, (c) youthful, silly and fun, and (d) ethnically specific.

In pharmaceutical methods for smoking cessation therapy, one objective is to deliver the sensations of the cigarette by a non-inhalation route, hence avoiding the toxic effects of tar and carbon monoxide in the cigarette smoke. Compounds of this invention may substitute for the menthol sensation of mentholated cigarettes by providing a prolonged refreshing feeling in the oral cavity and upper airways. Furthermore, the inventive compounds may be combined with nicotine or lobelline, or other nicotine receptor agonists, to form a product designed to both reduce the harsh taste of the nicotine, and to mimic the cooling and the central stimulant effects of a mentholated cigarette. The delivery methods may be a lozenge, chewing gum, spray, aerosol, syrup, gargle, mouthwash, film, or a candy designed for both cooling of the mucous membranes of the mouth and oropharynx and for buccal absorption of the nicotine.

### Use in Long-Acting NA CE for Nausea, Dysphagia, and Malaise

It has been known for some time that condiments with special flavors will counteract nausea, dysphagia, hyperphagia, and malaise from various ailments. For example, ginger flavored drinks or candy can counter the nausea of motion sickness in some individuals. The NACE compounds can also be utilized for such applications, and as a method of appetite suppression.

### Use in Long-Acting NACE for Heat Stress and Fever

Heat stress is defined as any combination of work, airflow, humidity, air temperature, thermal radiation, or internal body condition that strains the body as it tries to regulate its temperature. Fever occurs when heat production exceeds heat loss and oral or rectal and ear temperatures rises beyond 37.8°C and 38.3°C, respectively. When the strain to regulate body temperature exceeds the body's ability to adjust, heat stress can impair work performance, morale, mental alertness, increase the risk of accidents, and lead to heat-related illnesses. It has been found that oral doses of NACE compounds, usually more than 5 mg, will produce cooling sensations in the upper chest. These effects may originate from temperature sensors in the upper alimentary tract. Thus, delivery of NACE compounds into the body and onto thermoreceptors for cooling may be a method for coping and relieving the problems of fever and heat stress.

### Delivery to Target and Utility of N-Alkylcarbonyl-Amino Acid Esters

In formulating topical and oral compositions, the long-acting NACE compound may be incorporated into a vehicle that by itself may be inert or may contain other active ingredients.

Suitable formulations, for example, include compositions such as liquids, aerosols, powders, pastes, lotions, liniments, creams and ointments, and cosmetic preparations. A wide variety of vehicles will be suitable, depending upon the particular product involved, such vehicles including solids, liquids, emulsions, foams and gels. Typical vehicles include oils and fats such as hydrocarbon oils, polyhdric alcohols, calcium or magnesium stearate, fatty acid esters, long chain alcohols and silicone oils; finely divided solids such as starch or talc; low-boiling hydrocarbons; gums and natural or synthetic resins.

Suitable formulations for the oral cavity and throat, for example, include compositions such as liquids, powders, tablets, films or pastes. Lozenges (which are also called cough drops, troches, or pastilles) are pharmaceutical dosage forms used to treat tissues contained within the oral cavity and throat. A typical lozenge is composed predominantly of an inert vehicle, carrier, or diluent. A medicinal agent is interspersed within this carrier. The lozenge will dissolve when placed in the oral cavity thereby releasing the medicinal agent so that it may come in contact with the tissues of the mouth and throat. A typical diluent, carrier, or vehicle may be a "polyhydric alcohol" construed as describing the following substances: xylitol, mannitol, sorbitol, maltitol, isomaltitol, maltotriitol, lactitol, and β-linked-glucopyranasido-sorbitol. Flavoring agents such as the sweeteners, aspartame, sucralose, or alitame, may be added to mask any tastes. The mix is granulated to a uniformly dispersed blend; dispersing agents, anti-caking agents, and lubricants may be added; and the mixture is then compressed to form tablets for oral administration. Alternatively, the active ingredient may be put in a chewing gum formulation. These methods are familiar to those skilled in art and are described, for example, in US Patent No. 5,322,694 (David Sixsmith, Pharmaceutical Lozenges) and US Patent No. 5,846,557 (Eisenstadt et al. Chewing gum containing cough suppressing agent).

The duration of action of the active preparation may be further enhanced and localized at its sites of action (for example, the oropharynx) by the incorporation of mucoadhesive or bioadhesive agent. Such mucoadhesives or bioadhesives are, for example, described in US Patent No. 6,638,521 (D.J. Dobrozsi: Oral liquid mucoadhesive compositions) and US Patent No. 6,562,363 (J. Mantelle et al., Bioadhesive compositions and methods for topical administration of active agents). Typical adhesive molecules are polymers of sugars, alcohols, vinyl pyrrolidine, cellulose and the like. Dissolution of solid active ingredients in the oral cavity may sometimes be impeded by chewing or swallowing of the lozenge, or by the degree of hydration in the mouth. A liquid formulation for delivery may therefore be preferable. The NACE compounds are readily soluble (sometimes after warming) in aqueous solutions containing polyhdric alcohols, cyclodextrins, sugars and the like. These liquids, after sterilization by filtration, may be combined with preservatives, flavoring agents, solvents, and then dispensed from a reservoir type of storage container (e.g., a plastic container with a dropper type of opening) or from unit dose containers such as are readily available commercially. For example, Unicep Corporation in Sandpoint, Idaho, USA, has unit-dose contract packaging methods for volumes of 0.3 to 0.5 mL. A 2 to 20 mg/mL dose of a NACE compound would be an ideal form of unit dosage at these volumes of delivery. Alternatively, the NACE compound may be delivered with a nebulizer, a mouth-sprayer, or a hand-pump type of broncho-inhaler such as is well-known to those skilled in the art.

In practice, the long-acting NACE compound may also be applied onto the skin using a towelette that is of a construction sufficient or adapted to deliver the NACE compound to the skin. Thus, the desired NACE compound is suspended, dissolved, and/or dispersed so as to be in contact with the towelette. Suitable towelettes include a pad that may be of woven or nonwoven material usually in a unit dispenser. The wiping of the towelette or pad across skin results in delivery to the skin of dermatologically active ingredient(s), meaning that the skin is substantially medicated. Other drugs, cosmeceuticals, herbal medicines, traditional medicines, and active cosmetic ingredients suitable for topical human use may also be incorporated into the towelette.

It is contemplated that long-acting NACE compound may be incorporated into towelettes for treating the ageing skin; to treat the skin discomforts of acne, sunburn, fever, hyperthermia, fungal infections, yeast infections, rosacea, photodamaged skin; to reduce the discomforts of treatments for hyperpigmented skin, eczema, allergic or contact dermatitis, seborrheic dermatitis, mucositis, erythema, or psoriasis; and to be included with other dermatologic agents such as carboxylic acids, antibiotics, keratolytic agents or combinations thereof. In order to achieve a prolonged perfect cool, the long-acting NACE compound may also be combined with icilin, a cooling agent that acts at a molecular site that is distinct from the menthol site. Preferably, formulations are prepared with synthetic compounds that are at least 95 to 99% pure by standard analytical tests of homogeneity. The ability of long-acting NACE compound to impart cooling and refreshment in a towelette without sting, burn or irritation (especially to the eyes), is an advance over current technology on cooling agents. Known towelettes frequently contain SD Alcohol (specially denatured alcohol; usually ethanol, isopropyl alcohol or methanol), which is present as a solvent and/or a cooling agent. Alcohol produces cooling when it abstracts heat from its environs during evaporation. The drawback of using short-chain carbon-alcohols in such formulations is that the alcohol dehydrates tissues and causes irritation. When such a towelette is used near the eyeball, the alcohol vapors irritate the eye surface. Similarly, menthol, camphor, eucalyptol, and other ingredients added to towelettes to produce fragrance and cooling also irritate the skin and eyes.

In one embodiment, a long-acting NACE compound is carried by a towelette, which, for example, when applied to the face, will be especially valuable in counter-acting fatigue and to produce alertness and increased vigilance; for example, to combat tiredness from long car journeys or work in a hot environment.

### Summary of Experimental Results from Bioassays

The principal findings from experiments performed on the skin are summarized in Table 1. The beneficial effects of the long-acting NACE compounds are the long duration of action in the absence of significant eye irritation.

| Table 1 | | | | | |
|---|---|---|---|---|---|
| Summary Comparison of Unique Properties of Long-Acting NACE Compounds with Other Compounds | | | | | |
| Chemical Class | Cooling on tongue | Cooling on skin of face | "perfect" cooling experience | Eye Irritancy | Acts for >1 hour at M 40 mM |
| Long-acting NACE | yes | yes | yes | no | yes |
| non-NACE carboxamides | yes | variable | no | yes | no |
| (-)-menthol | yes | yes | no | yes | no |
| SD alcohol | no | yes | yes | yes | no |

### Chemical Synthesis

### Synthesis of N-Alkylcarbonyl-Amino Acid Esters

Synthesis of the NACE compounds may be achieved by reaction of the free amine with the appropriate acid chloride, usually in the presence of a suitable acceptor for hydrogen chloride, which could be an excess of the free amine or another base, e.g., triethylamine.

The reaction is typically carried out in a suitable organic solvent, but, depending on the reactivity of the acid chloride, may also be carried out in a mixed aqueous/organic solvent system, in which case a convenient base is sodium bicarbonate.

The synthesis commences with the reaction of a carbonyl chloride with the appropriate amino acid ester derivative. The acid chloride, or carbonyl chloride, may be obtained by many methods known to the art. As an example, (1R,2S,5R)-2-isopropyl-5-methylcyclohexane carbonyl chloride is prepared from (-)-menthol, via the following route: first, reaction with zinc chloride in hydrochloric acid to prepare 2-isopropyl-5-methylcyclohexyl chloride; next preparation of the Grignard reagent and carbonation to yield 2-isopropyl-5-methylcyclohexane carboxylic acid; and finally reaction with thionyl chloride to yield the carbonyl chloride.

In order to avoid problems with the purification of the NACE compound, it is advantageous to ensure that the thionyl chloride used for the preparation of the acid chloride is double-distilled before use. Also, depending upon the NACE compound, it may be advantageous to run the reaction with a slight excess of the acid chloride, or at elevated temperatures in order to avoid traces of free amine in the final product.

Such methods of adjusting the reaction to encourage conversion and/or to avoid certain impurities are well known to those skilled in the art.

Many substituted amino acid esters may be obtained from commercial sources such Sigma-Aldrich Corp., St. Louis, MO, USA. For example, sarcosine ethyl ester, β-alanine ethyl ester, R- or S- amino butyrolactone, and L- or D-alanine methyl ester, are listed in the 2003-2004 Aldrich Catalog. The precursor, D-alanine ethyl ester is available from Indofine Chemicals, Co., Hillsborough, NJ. The acid chloride is reacted with the appropriate amino acid ester to form the NACE compound.

### Synthesis of 2-Isopropyl-5-methyl-cyclohexanecarbonyl-L-Hsl (also known as: (1R,2S,5R)-2-isopropyl-5-methyl-cyclohexanecarboxylic acid (S)-(2-oxo-tetrahydro-furan-3-yl)-amide):

*(S)*-α-amino-γ-butyrolactone hydrochloride was obtained from Aldrich Chemical Co., 500 mg was dissolved in 18 mL diethyether and 1.5 mL double-distilled water. A pinch of the catalyst diaminopyrimidine was added. 0.68 mL of p-menthoyl chloride was then added dropwise, followed by 1.02 mL of triethylamine. The mixture was stirred overnight at room temperature. The precipitate was dissolved with ethyacetate, washed with double-distilled water and dried over sodium sulfate. The organic phase was then evaporated under reduced pressure to yield the final product, which crystallized at room temperature. The expected molecular mass was then confirmed by mass spectroscopy and the absorption spectrum by nuclear magnetic resonance.

### Synthesis of 2-Isopropyl-5-methyl-cyclohexanecarbonyl-D-Ala methyl ester (also known as: (R)-2-[((1R,2S,5R)-2-isopropyl-5-methyl-cyclohexanecarbonyl)-amino]-propionic acid methyl ester):

D-Ala methyl ester hydrochloride was obtained from Aldrich Chemical Co., 1.0 g was dissolved in 28 mL diethyether and 1 mL double-distilled water and cooled to 0°C. A pinch of the catalyst diaminopyrimidine was added. 1.62 mL of p-menthoyl chloride was then added dropwise, followed by 2 mL of triethylamine. Clumps of white precipitates appeared in the mixture, which was stirred overnight at room temperature. The precipitate was dissolved with ethyl acetate, washed with double-distilled water, and dried over sodium sulfate. The organic phase was then evaporated under reduced pressure to yield the final product (2 g), which crystallized at room temperature. The expected molecular mass was then confirmed by mass spectroscopy and the absorption spectrum by nuclear magnetic resonance.

### Synthesis of 2-Isopropy/-5-methy/-cyc/ohexanecarbony/-D-A/a methyl ester (also known as: (R)-2-[((1R,2S,5R)-2-isopropyl-5-methyl-cyclohexanecarbonyl)-amino]-propionic acid ethyl ester):

D-Ala ethyl ester hydrochloride was custom ordered from Indofine Chemical Co. To a stirred suspension of HCl.H-D-Ala-OEt (11.6 g; 71.6 mmol) in 100 mL of dry EtOAc, 21.14 mL (143.2 mmol) of Et₃N were added and reaction mixture was cooled to 0°C. 13.2 g (65.14 mmol) of p-menthoyl chloride was added and solution was stirred at 0°C for 1 hour and at room temperature for additional 2 hours. The reaction mixture was diluted with EtOAc to 300 mL, washed with 1 N HCl, 5% NaHCO₃, and water, and dried (Na₂SO₄). The solvent was removed and the residue was crystallized in Et₂O - hexane mixture. The purity of this initial product, by HPLC, was about 93%. For additional purification, the product was dissolved in 100 mL of isopropanol and 100 mL of distilled water was added. Isopropanol was evaporated and precipitate was filtered off and dried in vacuum. The final product purity by HPLC was >96%. Yield 14.2 g (76%). Melting point 99-103°C.

### Bioassay Procedures

The long-acting-NACE compounds are mostly white crystalline solids at room temperature, with the exception of the Sar Et Ester derivative, which was an oily liquid at room temperature.

For bioassay on the skin, approximately 30 mg was stirred and dissolved in 3 g of warm liquid Aquaphor® ointment to a yield a 40 mM (∼1% wt/vol) ointment. The exact amount was adjusted so that the molar equivalent was about 35 to 40 mM. After cooling, 40 to 70 mg of the solid ointment was placed on the tip of a plastic stick and applied to the skin above the upper lip, on the philtrum, and lateral to the philtrum, up to the nasolabial folds, of test subjects and the onset and duration of cooling sensations noted.

The intensity of the subjective skin sensation was rated as 0, 1, 2 or 3 with: 0 as no change; 1 as slight coolness, cold, or tingling; 2 as clear signal of coolness, cold, or tingling; and 3 as robust cooling or cold. The intervals for recording sensations were 5 to 10 minutes, until two successive zeroes were obtained. The results (shown in Figures 1 and 2) are averaged values of 4 to 6 separate trials in the same individual. The data are plotted using SigmaPlot® (Systat Software, Point Richmond, CA, USA) and a smoothing function with a negative exponential was used for analysis and statistical fit of the results.

For tests of irritancy near the eyes, the ointment was applied to the periorbital skin (upper and lower eyelids and on skin adjacent to the lateral canthus), and the subject asked if irritation is present or absent. The intensity of the eye sensation is not rated, but just noted as being present or absent.

The onset of drug action was taken as the time to reach 2 units of coolness intensity, and offset of drug action was the time when coolness intensity drops below 2, after previously surpassing 2 units. The duration of cooling action was defined as the offset time minus the onset time. An inactive compound is defined as one that did not exceed 2 units of cooling after application. The quality of the sensation was also noted: such as pure refreshing coolness, or if the sensation was accompanied by irritation (stinging or burning). The quality of the sensation was not rated for intensity.

### Study 1

A number of compounds were synthesized and tested with the results are shown in Figures 1 and 2. Test compounds were singly applied to the skin above the upper lips at a 40 to 70 mg dose of a 1 % wt/vol ointment.

In Figure 1, the duration of cooling effects of some known agents, e.g., (-)-menthol, WS-3 and WS-5 are 0.3, 0.3, and 0.5 hour, respectively, is relatively short compared to the NACE compounds, specifically, D-Hsl, D-Ala-OMe and D-Ala-OEt analogs with 1.3, 1.9, and 2.4 hours of cooling, respectively.

In Figure 2, the chemical features that influence duration of action are shown. WS-12, potent on TRP-M8 receptor assays, is not long-acting on the skin. The presence of a methyl group on the *N*-nitrogen of Sar-OEt increases potency. Similarly, the lactone ring in D-Hsl, the extra carbon on the α-carbon and D-configuration of D-Ala-OMe and D-Ala-OEt markedly increase the duration of action.

Additional data are shown in Table 2 and Table 3. Long-acting NACE derivatives that have a refreshing cool, without skin or eye irritancy, are identified by (*) in Table 2 and Table 3. The D-Ala and Hsl analogs are of special utility.

| Table 2 | | | | |
|---|---|---|---|---|
| 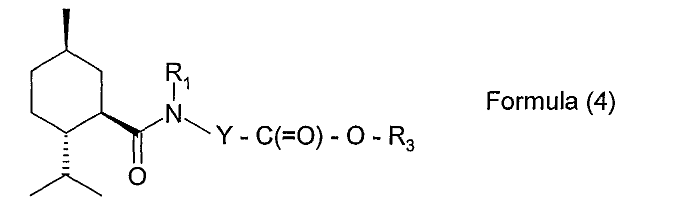 | | | | |

| Compounds | R₁ | Y | R₃ | Duration of Action On-Off (minutes) |
|---|---|---|---|---|
| | | | | |
| Gly Me Ester (WS-31) | H | -CH₂- | Me | 5 to 25 = 20 |
| Gly Et Ester (WS-5) (*) | H | -CH₂- | Et | 4 to 37 = 33 |
| | | | | |
| Sar Et Ester (*) | Me | -CH₂- | Et | 2 to 65 = 63 |
| L-Ala Et Ester | H | -CH(CH₃)- | Et | 8 to 52 = 44 |
| L-Ala Me Ester | H | -CH(CH₃)- | Me | 1 to 80 = 79 |
| D-Ala Me Ester (*) | H | -CH(CH₃)- | Me | 1 to 115 = 114 |
| D-Ala Et Ester (*) | H | -CH(CH₃)- | Et | 1 to 143 = 142 |
| β-Ala Et Ester | H | -CH₂-CH₂- | Et | 3 to 90 = 87 |

| Table 3 | | | |
|---|---|---|---|
| 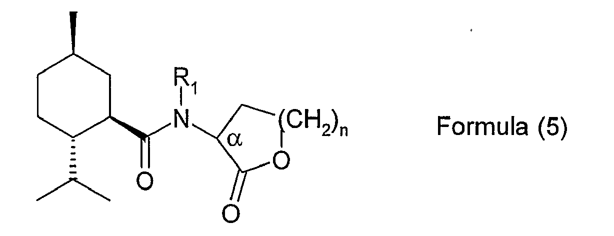 | | | |

| Compounds | R₁ | n | Duration of Action On-Off (minutes) |
|---|---|---|---|
| D-Hsl (*) | H | 1 | 2 to 80* = 78 |
| L-Hsl (*) | H | 1 | 2 to 87* = 85 |
| racemic Hsl (*) | H | 1 | 2 to 86* = 84 |

| | | | |
|---|---|---|---|
| (*) denotes compounds with refreshing cooling and absence of skin irritation or eye irritation after facial skin or periorbital applications; a "perfect cool." (Gly = glycine; Sar = sarcosine; Ala = alanine; Hsl = homoserine lactone (also known as α-amino-butyro-γ-lactone)) | | | |

Examples of compounds that were inactive when tested on the skin of the upper lip at 40 mM are shown in Table 4 and Table 5. (Ser = serine; Glu = glutamic acid; Lys = lysine; Tyr = tyrosine; Val = valine; Leu = leucine.)

| Table 4 | | | | |
|---|---|---|---|---|
| 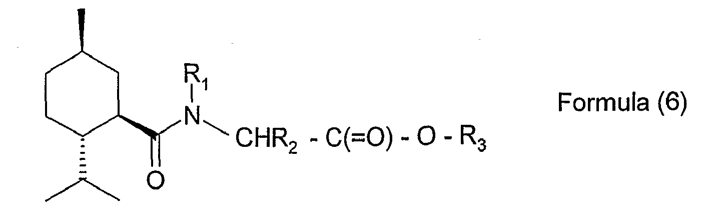 | | | | |

| Compounds | R₁ | R₂ | R₃ | Duration of Action On-Off (minutes) |
|---|---|---|---|---|
| Ser Et Ester | -H | -CH₂-OH | Et | not active |
| Glu(OMe) Me Ester | -H | -(CH₂)₂-C(=O)-OMe | Me | not active |
| Lys(Z) *t*-Bu Ester | -H | -(CH₂)₄-NH-C(=O)-O-CH₂-C₆H₅ | *t*-Bu | not active |
| Tyr Me Ester | -H | -CH₂-C₆H₄-4'-OH | Me | not active |
| L-Val Me Ester | -H | -CH-(CH₃)₂ | Me | not active |
| D-Val Me Ester | -H | -CH-(CH₃)₂ | Me | not active |
| Leu Me Ester | -H | -(CH₂)₂-CH-(CH₃)₂ | Me | not active |

| Table 5 | | |
|---|---|---|
| Compound | Structure | Duration of Action On-Off (minutes) |
| Pro Me Ester | 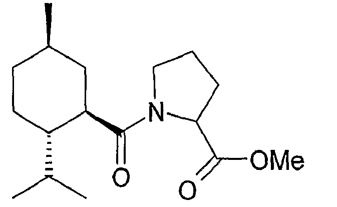 | not active |

Examples of *N*-alkyl- or aryl-cycloalkyl carboxamide compounds that have brief cooling action on the skin of the upper lip when tested at 40 mM are shown in Table 6.

| Table 6 | | |
|---|---|---|
| 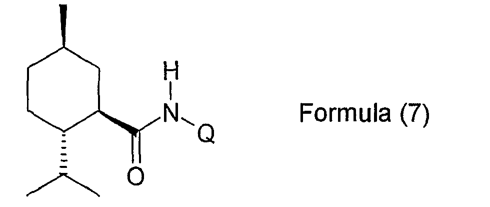 | | |

| Compound | Q | Duration of Action On-Off (minutes) |
|---|---|---|
| WS-3 | ethyl -CH₂CH₃ | 5 to 23 = 18 |
| WS-10 | isopropyl -CH(CH₃)₂ | 6 to 25 = 19 6 to 25 = 19 |
| WS-34 | sec-butyl -CH(CH₃)CH₂CH₃ | inactive |
| WS-14 | *t*-butyl -C(CH₃)₃ | 15 to 23 = 8 |
| WS-11 | 1',1'-dimethyl-2'-hydroxyethyl -C(CH₃)₂CH₂OH | 8 to 18 = 10 |
| WS-12 | 4'-methoxyphenyl -C₆H₅-4'-OMe | 13 to 38 = 25 |
| CPS-309 | 2',4'-dimethoxyphenyl -C₅H₄-2',4'-di-OMe | 13 to 50 = 37 |
| CPS-306 | 4'-ethoxyphenyl -C₆H₅-4'-OEt | inactive |
| CPS-310 | 4'-*n*-propoxyphenyl -C₆H₅-4'-*n*-OPr | inactive |
| CPS-308 | 4'-*n*-butoxyphenyl -C₆H₅-4'-*n*-OBu | inactive |

### Study 2

### Physical Properties and Tongue Thresholds for Cooling of Various Compounds

The octanol/water partition coefficients (given in log P units) for a number of compounds were determined and are shown in Table 7, together with the threshold for cooling on the tongue tested at 40 mM. As can be seen from this data, there is no correlation between tongue cooling and the duration of cooling. A longer duration of action was not precisely matched with log P values.

| Table 7 | | | | |
|---|---|---|---|---|
| Compound | Molecular Weight | Threshold tongue (µg) | Log P | Duration of Skin Cooling |
| Gly Me Ester (WS-31) | 255.4 | 0.6 | 2.4 | 0.3 hour |
| Gly Et Ester (WS-5) | 269.4 | 0.2 | 2.9 | 0.5 hour |
| Sar Et Ester | 283.4 | 0.8 | 2.9 | 1.0 hour* |
| L-Ala Et Ester | 283.4 | 0.4 | 3.1 | 0.7 hour |
| L-Ala Me Ester | 269.4 | 0.6 | 2.8 | 1.3 hour* |
| D-Ala Me Ester | 269.4 | - | 2.8 | 1.9 hour* |
| D-Ala Et Ester | 283.4 | - | 3.1 | 2.4 hour* |
| β-Ala Et Ester | 283.4 | 1.5 | 3.1 | 1.4 hour* |
| D-Hsl | 267.4 | - | 2.5 | 1.3 hour* |
| L-Hsl | 267.4 | - | 2.5 | 1.4 hour* |
| racemic Hsl | 267.4 | - | 2.5 | 1.4 hour* |
| WS-3 | 211.3 | 0.2 | 3.7 | 0.3 hour |
| WS-10 | 225.4 | 0.4 | 4.1 | 0.3 hour |
| WS-34 | 239.4 | 0.7 | 4.6 | not active |
| WS-14 | 239.4 | 0.4 | 4.5 | 0.1 hour |
| WS-11 | 255.4 | 0.3 | 2.9 | 0.2 hour |
| WS-12 | 289.4 | 0.2 | 5.3 | 0.4 hour |
| CPS-309 | 319.4 | - | 5.0 | 0.6 hour |
| L-Ser Et Ester | 285.4 | - | 1.8 | not active |
| L-Val Me Ester | 297.4 | - | 4.2 | not active |
| D-Val Me Ester | 297.4 | - | 4.2 | not active |
| Glu(OMe) Me Ester | 341.4 | - | 2.2 | not active |
| L-Leu Me Ester | 311.5 | - | 4.3 | not active |
| L-Pro Me Ester | 295.4 | - | 3.3 | not active |
| L-Lys(Z) *t*-Bu Ester | 502.7 | - | 5.9 | not active |
| L-Tyr Me Ester | 364.2 | - | 3.7 | not active |
| CPS-306 | 303.4 | - | 5.8 | not active |
| CPS-310 | 317.5 | - | 6.3 | not active |
| CPS-308 | 331.5 | - | 6.9 | not active |

| | | | | |
|---|---|---|---|---|
| (*) denotes compounds that fulfill the criteria of being a long-acting NACE compound (i.e., > 1 hour duration of action). | | | | |

Again, the D-Ala Me ester and Hsl analogs are of special note because of the absence of significant skin or ocular irritation.

### Study 3

2-Isopropyl-5-methyl-cyclohexanecarbonyl D-alanine methyl ester was dissolved in warm 10% propylene-glycol/90% distilled water solution to give a concentration of either 0.1 or 0.5% wt/vol (1 or 5 mg/mL). Six to seven milliliters of these solutions were then applied with a pipette onto a paper napkin (Luncheon Napkins, Kirkland Signature brand from Costco, Inc.) The napkin was 1-ply with a 30.4 x 29.5 cm dimension. Each napkin was then hermetically sealed in a plastic envelope (Foodsaver by Tilia). On two separate occasions involving golfing trips to Los Angeles, California, USA, where air temperatures exceeded 332°C (90°F), these towelettes were used to wipe the face. Pleasant cooling sensations were obtained that lasted for about 10 minute for the 0.1 % concentration towelette and about 60 minutes for the 0.5% towelette. No eye irritation was observed with either concentration.

### Study 4

A 1% preparation of 2-isopropyl-5-methyl-cyclohexanecarbonyl D-alanine methyl ester in Aquaphor® ointment was applied bilaterally to the periorbital area of four individuals. Onset of coolness on the skin was noted with 1 minutes and lasting for an average of 30 minutes. No irritation of the eye surfaces was noted. Surprisingly, all four individuals noted an alerting effect and an ability to focus and see more clearly. This alerting effect lasted for about one hour. This experiment was then repeated three days later in the evening with the same individuals whose occupations required daylong activities before a computer screen. Again, application of the ointment about the periorbital area was reported to relieve fatigue, enhance visual acuity, and to increase attention span and focus. Sensations of refreshment and improved mood were also noted. By contrast, tests with other carboxamides, WS-3, WS-23, WS-11, and WS-14, under similar conditions, showed that they produced significant skin and eye irritation and did not enhance skin coolness or provide satisfactory refreshment.

### Study 5

A twenty-year-old female was treated with Tazorac® (tazarotene topical gel) for acneiform dermatitis on her cheeks. After using these medications for several days, she noted redness, itching, and uncomfortable burning sensations on the skin where these medications had been placed. A 1.5% preparation of 2-isopropyl-5-methyl-cyclohexanecarbonyl D-alanine methyl ester in Aquaphor® ointment was then applied to the sites of irritation. Sensations of coolness on the skin were noted within 5 minutes and lasting for an average of 30 minutes. The discomfort of the irritated skin was abated within five minutes and the subject felt better.

### Study 6

### Safety Profile of Active Ingredients

Many of the preferred compound of Formula (1) may be viewed as having three components: a (1R,2S,5R)-2-isopropyl-5-methylcyclohexane carbonyl moiety; D-alanine; and an alkyl ester.

The first component is found in WS-3, a cooling agent generally recognized as safe (GRAS) and permitted for use in cosmetics, toiletries and confectionery. WS-3 is commercially available from Qaroma, Baytown, Texas, USA at 99.5+% purity and from Millennium Specialty Chemicals at 99-100% purity. The Flavor and Extract Manufacturer Association (FEMA), GRAS Code for WS-3 is 3455 and its safety profile is fully documented.

The second component, D-alanine, is part of the artificial sweeteners aspartame and alitame, and hence has been evaluated for safety. D-Alanine is incorporated into the cell walls of bacteria but not humans. C¹⁴-labelled D-alanine is metabolized to carbon dioxide and water in rats.

The alkyl esters may be hydrolyzed from the amino acid to form the alcohol and then further degraded to carbon dioxide and water. The alkyl esters do not have toxic potential. Thus, these molecules have a good safety profile.

### Study 7

### Comparison of D- and L-Compounds

The L-homoserine lactone, (1R,2S,5R)-2-isopropyl-5-methyl-cyclohexane carboxylic acid (S)-(2-oxo-tetrahydro-furan-3-yl)-amide (an analog of compounds of Formula (2)), bears some resemblance to the *N*-acylhomoserine lactone family of molecules secreted by Gram-negative bacteria. These "quorum-sensing" signal molecules constitute one of the few mechanisms by which bacteria can communicate with each other. Although not fitting the exact structural requirements for quorum-sensing activity, it is likely that the L-homoserine lactone analog will face severe toxicology scrutiny for pharmaceutical applications.

The D-homoserine lactone does not possess quorum-sensing activity but the D-Hsl starting material is likely to be expensive for manufacture of the final product in bulk quantities. Thus, the D-homoserine lactone compound, (1R,2S,5R)-2-isopropyl-5-methylcyclohexane carboxylic acid (R)-(2-oxo-tetrahydro-furan-3-yl)-amide (an analog of compounds of Formula (2)) may be used as a cough inhibitor, but it is more expensive than the compounds of Formula (1).

The compounds of Formula (1) and (2) are white crystalline solids at room temperatures. These compounds were tested as such, or mixed 1:1 with powdered or granulated sugar. The test substances were applied at 1 to 5 mg on the mid-point of the dorsal surface of the tongue using a metal spatula or a plastic applicator. After depositing the test substance on the tongue, the subject was instructed to keep the mouth closed and allow the fluids of the mouth to inter-mix with the test substance and to distribute it to the back of the mouth (oropharynx). The presence and duration of subjective sensations from the mouth was then noted.

In normal subjects, the effects of the D-Ala methyl and ethyl ester compounds falling within Formula (1) ((R)-2-[((1R,2S,5R)-2-isopropyl-5-methyl-cyclohexanecarbonyl)-amino]-propionic acid methyl or ethyl ester)) were examined. Three doses of 1, 2 and 4 mg per application were examined and compared to a similar dose of the L-Ala methyl or ester analog ((S)-2-[((1R,2S,5R)-2-isopropyl-5-methyl-cyclohexanecarbonyl)-amino]-propionic acid methyl or ethyl ester). At the 2 or 4 mg dose, all compounds produced pleasant and robust cooling sensations on the tongue, extending down to the back of throat, and lasted for about 30 minutes and longer. The onset effect was usually within 30 seconds of application.

The D-analogs were clearly more active than the L-equivalent. At the higher doses, there was also cooling sensations of chest, most likely due to activation of receptors in the esophagus and the radiation of such sensations into the thorax. This particular sensation may be aversive in a cold environment. Hence, compounding the active ingredient with an adhesive molecule to localize the drug action in the throat may be advantageous.

Based on an analysis of the coolness-duration response (area under the curve), the D-analogs were at least three times more potent and refreshing than the L-analogs. Menthol tested under the same conditions produced cooling primarily in the nasopharynx and less in the oral cavity. Also, the effects of menthol were short-lived, on the order 8 to 12 minutes. All analogs were accompanied by a slightly bitter taste similar to that of (-)-menthol.

D-, L-, and racemic Hsl analogs falling within Formula (2) ((1R,2S,5R)-2-isopropyl-5-methyl-cyclohexanecarboxylic acid (R/S)-(2-oxo-tetrahydro-furan-3-yl)-amide) were tested at 2 mg per dose. These compounds also produced robust and prolonged cooling at the back of the throat. These compounds had an ester-like taste that was pleasant and aromatic.

Under the same test conditions, WS-5 and the sarcosine ethyl ester analog, both without the D-Ala steric configuration, also had cooling properties, but the duration of action was less than 10 minutes and there was some sensory irritation with these compounds.

### Study 8

Two males, both aged 60 years, were inveterate cigar smokers consuming 3 to 5 cigars per day. They both had frequent bouts of dry, non-productive, hacking coughs. At night, there were episodes of insomnia characterized by difficulties in breathing, an oppressive pressure on the chest and throat, and subsequent awakening with sensations of choking. During the day, a frequent and persistent sensation was that of tickling, irritative feelings in the back of the throat that led to coughing.

In 20 trials, 10 trials per subject, two mg of 95%+ pure powder of a D-Ala ethyl compound falling within Formula (1) (R)-2-[((1R,2S,5R)-2-isopropyl-5-methyl-cyclohexanecarbonyl)-amino]-propionic acid ethyl ester) was placed on the mid-portion of the tongue of these two subjects. Both subjects reported relief from the desire to cough within 1 minute after application. Sensations of robust coolness in the back of the throat was noted and lasted for about 30 minutes. Applications of similar amounts of powdered sugar were not effective, but the placebo could be clearly distinguished from the active compound because of the absence of cooling sensations.

After application of test substance, the absence of irritation and itching at the back of the throat (cough signals) lasted for about 4 to 5 hours and the individual was observed not to cough for 6 to 8 hours. Surprisingly, after 4 applications in one individual and after 6 applications in the second individual, within a period of 2 days, the individuals noted that the desire to cough was removed for at least 5 days, in spite of continued smoking of cigars. This suggested that the test compound may have down-regulated the cough receptor mechanisms and permanently raised the threshold for cough stimuli. This therapeutic effect occurred when the test substance no longer elicited cooling sensations in the oropharynx.

### Study 9

A 61-year old female with a documented 31-year history of asthma attacks volunteered to test one of the compounds falling within Formula (1). She was extremely sensitive to agents such as tobacco smoke, dust, pollen, odors, down/woolen materials, house cleansing products, pets, and sudden change of air temperature, and reacted with violent coughing, mucus secretions, wheezing, choking, and shortness of breath. At the time of this trial, she was taking these medications for her condition: theophylline, Allegra®, Advair®, Intal®, and Singular®. In 5 trials, during a coughing spree, she placed one to two mg of 95%+ pure powder of a D-Ala ethyl ester falling within Formula (1) ((R)-2-[((1R,2S,5R)-2-isopropyl-5-methyl-cyclohexanecarbonyl)-amino]-propionic acid ethyl ester)) on the mid-portion of her tongue. She reported a soothing and cool sensation with a slight taste similar to Eucalyptus oil in her mouth and throat. Coughing stopped after 3 to 5 minutes and breathing was normal. She did not sense any build up of mucus or other side-effects. In three trials when the compound was applied at night, she slept through the night without being awakened by cough. Her husband remarked that, in over 30 years of marriage, this was the first time that he had seen an effective medication for her cough. She noted that the irritative signals of cough were absent in the presence of the applied chemical, but she still sensed a mechanical build up of secretions in her airways and could voluntarily cough up such secretions if necessary. She continues to use the test compound for management of her asthma, with the knowledge and consent of her physician. Surprisingly, her asthmatic coughing attacks have not recurred with any severity for one month. This effect suggests that one of the pathophysiological signs of asthma, airway hyper-reactivity, may be attenuated by the treatment.

### Study 10

A 74-year old male had adult-onset asthma for 20 years. Triggers for attacks were tobacco smoke, dust, and changes in air temperature. He used Foradil® (formoterol fumarate) and Asmanex® (mometasone fuorate) twice a day to control his asthma, and monitored his pulmonary function with a portable device for measuring peak expiratory flow. He had daily episodes of both productive and non-productive cough. In 5 trials, when he felt throat irritation and a desire to cough, placement of 1 to 3 mg of (R)-2-[((1R,2S,5R)-2-isopropyl-5-methyl-cyclohexanecarbonyl)-amino]-propionic acid ethyl ester) provided rapid relief and was described as soothing and removed "tightness" in the chest. Peak expiratory flow improved from 320 Umin to 370 Umin. In 3 episodes where the test substance was administered in the midst of a productive cough spell, no significant effects were observed and the subject said that the applied compound had been expectorated. The subjective response to the long-acting NACE compound was definitely favourable. The subject remarked that the best time to use the NACE compuound was after inhaling the crystalline Foradil® because this inhaled drug irritated his throat. Menthol lozenges were not effective for his airway discomfort because they only had mild anti-irritant actions and the sugar content of the lozenges interfered with his appetite for food.

The foregoing has described the principles, preferred embodiments, and modes of operation of the present invention. However, the invention should not be construed as limited to the particular embodiments discussed. Instead, the above-described embodiments should be regarded as illustrative rather than restrictive, and it should be appreciated that variations may be made in those embodiments by workers skilled in the art without departing from the scope of the present invention.

## Claims

1. A compound of Formula (1): 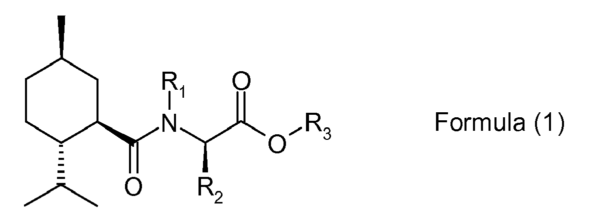 wherein:
R₁ is independently hydrogen or methyl;
R₂ is independently C₁ to C₂ alkyl; and
R₃ is independently C₁ to C₄ alkyl;
or a salt or solvate thereof, for use in the treatment of diseases or conditions mediated by activation of the TRP-M8 receptor.

2. A compound of Formula (1) or a salt or solvate thereof for use according to claim 1, wherein the disease or condition is nostril and nasal or upper airway discomfort from breathing obstruction.

3. A compound of Formula (1) or a salt or solvate thereof for use according to claim 2 wherein the discomfort is caused by rhinitis, asthma, bronchitis, emphysema and chronic obstructive pulmonary diseases, dyspnea, sleep apnea and snoring.

4. A compound of Formula (1) or a salt or solvate thereof for use according to claim 1 wherein the condition is involuntary cough.

5. A composition comprising a compound of Formula (I) or a salt or solvate thereof, adapted to be delivered as a liquid.

6. A composition according to claim 5 wherein the liquid is delivered as a mist or spray.

7. A composition according to claim 5 in unit dosage form.

8. A composition according to claim 7 wherein the unit dose is 2 to 20mg/mL.

9. A composition according to claim 5 wherein the composition is contained in a nebulizer, mouth-sprayer or inhaler.

10. A composition comprising a compound of Formula (I) or a salt or solvate thereof, in combination with a β-adrenoreceptor agoinst, an anti-inflammatory glucocorticosteroid or a muscarinic receptor antagonist.

11. A compound of Formula (I) or a salt or solvate thereof, for use in the treatment of cough to restrict contagion and spread of pathogenic respiratory viruses.

12. A composition comprising a compound of Formula (I) or a salt or solvate thereof, in combination with a nicotine receptor agonist.

13. The compound for use according to claim 1, or a salt or solvate thereof, wherein the TRP-M8 receptors to be activated are located on the glossopharyngeal nerve.

14. The compound for use according to claim 13 or a salt or solvate thereof, wherein the TRP-M8 receptors are located on the sensory nerve endings of the glossopharyngeal nerve.

15. The compound for use according to claim 13 or 14 or a salt or solvate thereof, wherein the glossopharyngeal nerve is in the naso or oropharynx.
